# EUROPÄISCHE PATENTSCHRIFT

(11) **EP 1 606 420 B1**
(45) Veröffentlichungstag und Bekanntmachung des Hinweises auf die Patenterteilung: **03.08.2011**
(21) Anmeldenummer: 04721892.0
(22) Anmeldetag: 19.03.2004
(51) Int. Cl.: C12Q 1/68

(54) **VERFAHREN UND KIT ZUM SPEZIFISCHEN NACHWEIS VON M. TUBERCULOSIS**
METHOD AND KIT FOR A SPECIFIC DETECTION OF M.TUBERCULOSIS
PROCEDE ET KIT POUR DETECTER M. TUBERCULOSIS DE MANIERE SPECIFIQUE

(30) Priorität: 20.03.2003 DE 10313791
(43) Veröffentlichungstag der Anmeldung: 21.12.2005
(73) Patentinhaber: Qiagen Hamburg GmbH, 22767 Hamburg (DE)
(72) Erfinder: BANGE, Franz-Christoph, 30171 Hannover (DE)
(74) Vertreter: Weber, Martin
(86) Internationale Anmeldenummer: PCT/EP2004/002911
(87) Internationale Veröffentlichungsnummer: WO 2004/083459

(56) Entgegenhaltungen:
- US-B1- 6 294 328
- DATABASE EMBL [Online] IBE; 2. Mai 2001 (2001-05-02), FLEISCHMANN, RD ET AL.: "Mycobacterium tuberculosis CDC1551, section 83 of 280 of the complete genome" XP002286366 gefunden im WWW.EBI.AC.UK Database accession no. AE006997
- COLE S T: "DECIPHERING THE BIOLOGY OF MYCOBACTERIUM TUBERCULOSIS FROM THE COMPLETE GENOME SEQUENCE" NATURE, MACMILLAN JOURNALS LTD. LONDON, GB, Bd. 393, 11. Juni 1998 (1998-06-11), Seiten 537-544,1, XP002144873 ISSN: 0028-0836
- STERMANN M ET AL: "Polymorphic nucleotide within the promoter of nitrate reductase (NarGHJI) is specific for Mycobacterium tuberculosis" JOURNAL OF CLINICAL MICROBIOLOGY 01 JUL 2003 UNITED STATES, Bd. 41, Nr. 7, 1. Juli 2003 (2003-07-01), Seiten 3252-3259, XP002286365 ISSN: 0095-1137
- LACHNIK J ET AL: "Rapid-cycle PCR and fluorimetry for detection of mycobacteria" JOURNAL OF CLINICAL MICROBIOLOGY, WASHINGTON, DC, US, Bd. 40, Nr. 9, September 2002 (2002-09), Seiten 3364-3373, XP002252656 ISSN: 0095-1137
- SREEVATSAN SRINAND ET AL: "Restricted structural gene polymorphism in the Mycobacterium tuberculosis complex indicates evolutionarily recent global dissemination" PROCEEDINGS OF THE NATIONAL ACADEMY OF SCIENCES OF USA, NATIONAL ACADEMY OF SCIENCE. WASHINGTON, US, Bd. 94, Nr. 18, September 1997 (1997-09), Seiten 9869-9874, XP002206250 ISSN: 0027-8424

## Beschreibung

Die vorliegende Erfindung betrifft Verfahren und Mittel zum spezifischen Nachweis von *Mycobacterium tuberculosis (M. tuberculosis)* in klinischen Material, wobei insbesondere die Unterscheidung zwischen *M. tuberculosis* und den anderen Mitgliedern des sogenannten *M.* tuberculosis-Komplexes, das sind *Mycobacterium bovis (M. bovis)*, *Mycobacterium bovis* BCG *(M. bovis* BCG*)*, *Mycobacterium africanum (M. africanum)* und *Mycobacterium microti (M. microti),* erreicht wird.

Gegenwärtig sind cirka 2 Milliarden Menschen weltweit mit dem Erreger der Tuberkulose, *M. tuberculosis,* infiziert, wobei jährlich 8 Millionen an Tuberkulose erkranken. Von den an Tuberkulose erkrankten versterben jährlich 3 Millionen. Damit ist die Tuberkulose weltweit zur Zeit die am häufigsten zum Tode führende bakterielle Infektionserkrankung. In den meisten Industrienationen besteht aufgrund der Gefährlichkeit dieser Erkrankung eine sofortige Meldepflicht nach Diagnose einer Tuberkulose, nicht zuletzt um so schnell wie möglich eine Ausbreitung in der Bevölkerung zu verhindern. In den letzten Jahren kann ein vermehrtes Auftreten von Tuberkulose sowohl in den Entwicklungsländem als auch in den Industrienationen beobachtet werden, wobei vor allem bei immunsupprimierten HIV-Patienten dadurch regelmäßig Todesfälle verzeichnet werden. Jedoch nicht nur wegen der allgemeinen Gefahr für große Teile der Weltbevölkerung, sondern auch wegen des inzwischen epidemieartigen Auftretens von mehrfachresistenten Bakterienstämmen (MDRTB) innerhalb oder außerhalb von Krankenhäusern wird die schnelle und eindeutige Diagnose zum Nachweis von *M*. *tuberculosis* gefordert.

Beim Menschen wird die Tuberkulose weltweit praktisch ausschließlich durch den humanen Tuberkuloseerreger *Mycobacterium tuberculosis* hervorgerufen. Davon abzugrenzen sind *Mycobacterium bovis (M. bovis), der Erreger der Rindertuberkulose, Mycobacterium bovis* (BCG), ein attenuierter Stamm von *M. bovis,* der zur Impfung gegen die Tuberkulose verwendet wird, sowie *Mycobacterium africanum (M. africanum)* und *Mycobacterium microti (M. microti).* Aufgrund ihrer nahen Verwandtschaft werden alle vorgenannten Spezies im sogenannten *M*. *tuberculosis-Komplex* zusammengefaßt. Davon wiederum abzugrenzen sind sogenannte nicht-tuberkulöse Mykobakterien. Davon existieren derzeit ca. 100 verschiedene Spezies, von denen wenigstens 30 Spezies in menschlichen Materialien wie Sputum, Stuhl, Urin etc. vorkommen und teilweise auch zu Infektionen bzw. Erkrankungen führen können. Ziel der gegenwärtigen Bemühungen auf dem Gebiet der Mykobakterien-Diagnostik ist daher nicht allein die Bereitstellung von Mykobakterien-Tests, welche es erlauben, eine Mykobakterieninfektion innerhalb kürzester Zeit in einem frühen Infektionsstadium zu erkennen, sondern vielmehr die Mitglieder des *M*. tuberculosis-Komplexes spezifisch zu identifizieren, und gleichzeitig *M. tuberculosis* von anderen Mitgliedern des M. tuberculosis-Komplexes zu unterscheiden.

Eine Identifizierung der Mitglieder des *M. tuberculosis*-Komplexes sowie die Unterscheidung zwischen *M. tuberculosis* und den anderen Mitgliedern des *M*. *tuberculosis-*Komplexes kann normalerweise durch Testen der Nitratreduktase-Aktivität und Niacin-Akkumulation der Mykobakterien erfolgen (z.B. Metchock B.G. et al. In: Manual of Clinical Microbiology. ASM Press, Washington DC, 1999: 399-437). Dabei zeichnet sich *M. tuberculosis* im Vergleich zu den anderen Mitgliedern des *M*. *tuberculosis*-Komplexes durch eine erhöhte Nitratreduktaseaktivität aus. Um den Nitratreduktase-Test durchzuführen, ist aber vor allem die Kultivierung der aus dem klinischen Material isolierten Mykobakterienstämme, insbesondere also tuberkulöser Erregerstämme im Labor erforderlich, was normalerweise allein in speziell für die Kultivierung hochinfektiöser Erreger ausgestatteten Labors möglich ist und wegen des langsamen Wachstums der Erreger mehrere Wochen dauert. Die Verfügbarkeit solcher Tests in der klinischen Routinediagnostik ist daher mit einen hohen labortechnischen und finanziellen Aufwand verbunden.

Alle bisher bestehenden Nachweismethoden werden als unbefriedigend und deshalb verbesserungswürdig angesehen. Aus dem Stand der Technik ist bisher kein für den klinischen Routineeinsatz geeignetes Verfahren bekannt, das sich insbesondere durch einfache Anwendung auszeichnet und mit dem es gelingt, die Mitglieder des *M*. *tuberculosis*-Komplexes spezifisch in klinischem Material wie Sputum, Bronchiallavage, Magensaft, Urin, Stuhl, Knochenmark, Blut oder in Biopsien nachzuweisen und gleichzeitig *M. tuberculosis* von den anderen Mitgliedern des *M. tuberculosis*-Komplexes zu unterscheiden.

Es ist bekannt, dass der Einsatz der Echtzeit-PCR ("Rapid-Cycle-PCR"), die beispielsweise mit einem lufttemperierten System ausgestattet ist und somit wesentlich geringere Transitionszeiten gegenüber einer herkömmlichen PCR aufweist, zu einer deutlich verminderten Zeit bis zum Nachweis von beispielsweise Mykobakterien mittels Amplfikation des isolierten genetischen Materials der Mykobakterien führt (Chäpin und Lauderdale, J. Clin. Microbiol. (1997) 35:2157-2159). Darüber hinaus stellen fluorimetrische Messungen bei der Anwendung farbmarkierter Hybridisierungssonden, insbesondere im Rahmen der Echtzeit-PCR, eine schnelle und empfindliche Methode zum Nachweis amplifizierter Genfragmente dar. Bislang ist es jedoch noch nicht gelungen, einen (Echtzeit-)PCR-Test bereitzustellen, mit dem die Mitglieder des *M*. *tuberculosis-*Komplexes spezifisch nachgewiesen werden, und gleichzeitig *M. tuberculosis* von den anderen Mitgliedern des *M. tuberculosis*-Komplexes unterschieden wird.

WO 03/016562 beschreibt Zusammensetzungen und Verfahren zur Detektion von Stämmern von *M. tuberculosis* mit multiplen Medikamenten-Resistenzen, welche Mutationen in Genen der *MUTT*-Familie aufweisen.

Vor diesem Hintergrund liegt die Aufgabe der vorliegenden Erfindung darin, insbesondere verbesserte Verfahren und Mittel bereitzustellen, welche im Wesentlichen einen besonders schnellen und gleichzeitig spezifischen Nachweis von den Mitgliedern des *M. tuberculosis*-Komplexes ermöglichen und mit denen es gleichzeitig möglich ist, *M. tuberculosis* von den anderen Mitgliedern des *M. tuberculosis*-Komplexes zu unterschneiden.

Erfindungsgemäß wird die Aufgabe durch den Gegenstand der Ansprüche 1 bis 30 gelöst.

Die vorliegende Erfindung betrifft insbesondere ein Verfahren zum spezifischen Nachweis von *M. tuberculosis* in klinischem Material, bei dem man
mikrobielle DNA aus dem klinischen Material extrahiert,
mindestens ein DNA-Fragment des Nitratreduktase-Operons von Mykobakterien, das heißt des narGHJI-Operons, welches Nitratreduktase codiert, aus der extrahierten DNA amplifiziert, wobei das amplifizierte DNA-Fragment oder eines der amplifizierten DNA-Fragmente eine Zielregion beinhaltet, welche mindestens einen für *M. tuberculosis* spezifischen DNA-Polymorphismus umfasst, der an Position - 215 in 5' zu 3' Leserichtung strangaufwärts des Translationsstartcodons GTG des narGHJI- Operons lokalisiert ist,
wobei die spezifische Hybridisierung des mindestens einen amplifizierten DNA-Fragments mit mindestens einer Hybridisierungssonde detektiert wird.

Unter *narGHJI*-Nitratreduktase-Operon wird erfindungsgemäß eine für *narGHJI-*Nitratreduktase kodierende Nucleinsäuresequenz verstanden, einschließlich der regulativen Sequenzen, insbesondere einschließlich seines Promotors. Bei dem für *M*. *tuberculosis* spezifischen Polymorphismus handelt es sich um einen gegenüber den Nucleinsäuresequenzen der anderen Mitglieder des *M. tuberculosis*-Komplexes, d.h. *M. bovis,* BCG, *M*. *africanum* und *M. microti,* spezifischen einzelnen Nucleotidaustausch (T->C), d.h. einen SNP (single nucleotide polymorphism).

In einer Ausführungsform der Erfindung wird in einem ersten Schritt mikrobielle DNA aus der Probe, die bevorzugt klinisches Material ist, extrahiert, und in einem weiteren Schritt mindestens ein DNA-Fragment des Nitratreduktase-Operons von Mykobakterien, das heißt des *narGHJI*-Operons, welches Nitratreduktase codiert, aus der extrahierten DNA amplifiziert, wobei das amplifizierte DNA-Fragment - oder eines der amplifizierten DNA-Fragmente (soweit in der biologischen Probe Vertreter mehrere Mycobakterien-Stämme vorliegen) - eine Zielregion (Target-Sequenz) beinhaltet, welche den mindestens einen für *M. tuberculosis* spezifischen DNA-Polymorphismus aufweist.

In einem weiteren Schritt wird die spezifische Hybridisierung des mindestens einen amplifizierten DNA-Fragments mit mindestens einer Hybridisierungssonde, das heißt Oligonucleotid, detektiert, wobei die Hybridisierungssonde eine Nucleotidsequenz umfasst, die vorzugsweise aus der Gruppe bestehend aus der in SEQ ID NO: 5 dargestellten Nucleotidsequenz, der komplementären Sequenz zu SEQ ID NO: 5, der in SEQ ID NO: 6 dargestellten Nucleotidsequenz und der komplementären Sequenz zu SEQ ID NO: 6 ausgewählt ist, und wobei der spezifische Nachweis von *M. tuberculosis* gegenüber anderen Mitgliedern des *M.* tuberculosis-Komplexes, das heißt vor allem *M. bovis,* BCG, *M. africanum* und *M. microti,* mittels Analyse der Schmelztemperatur der spezifischen Hybridisierung der Hybridisierungssonde mit dem amplifizierten DNA-Fragment des *narGHJI*-Operons, das heißt mittels Schmelzkurvenanalyse, erfolgt. Gemäß einer besonderen Ausführungsform weist die Sonde/weisen die Sonde/Sonden die vorgenannte/vorgenannten Nucleotidsequenz/Nucleotidsequenzen auf. Gemäß einer besonderen Ausführungsform werden die vorgenannten Hybridisierungssonden mit der Ankersonde gemäß SEQ ID NO: 4 oder der dazu komplementären Nucleotidsequenz kombiniert.

Der mindestens eine *M. tuberculosis*-spezfische DNA-Polymorphismus des *narGHJI*-Operons ist an Position -215 in 5' zu 3'-Leserichtung strangaufwärts des Translationsstartcodons GTG des *narGHJI*-Operons, das heißt in der Promotorregion, im Folgenden als *narGHJI*-Promotor bezeichnet, lokalisiert.

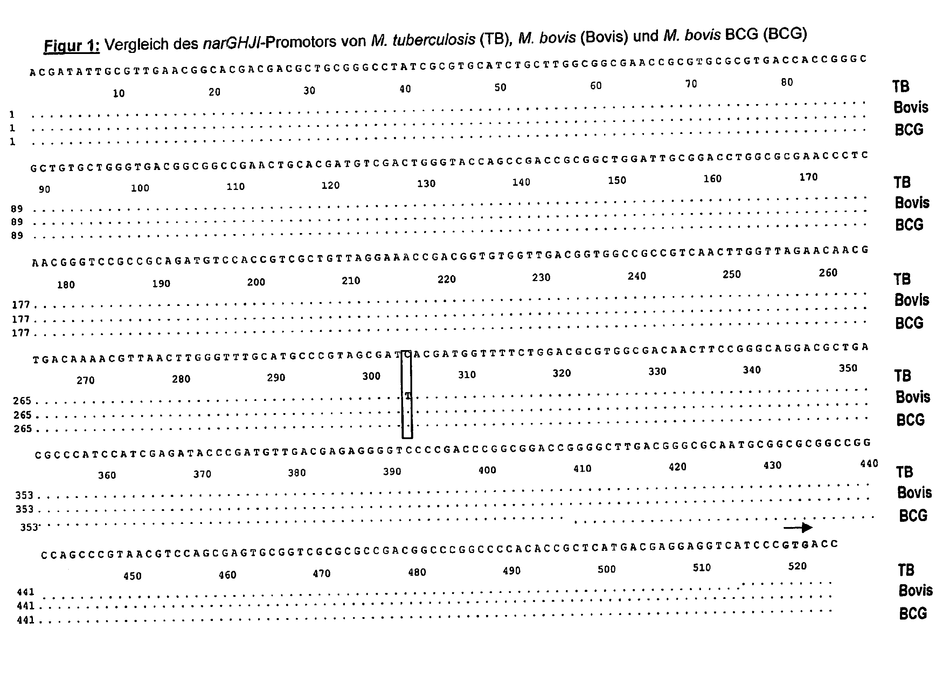

Es wurde überraschenderweise im Rahmen der vorliegenden Erfindung festgestellt, dass an der Stelle des 215ten Nucleotids in 5' zu 3'-Leserichtung strangaufwärts des Translationsstartcodons GTG des *narGHJI*-Operons, das heißt an Position -215 des *narGHJI*-Promotors, bei *M. tuberculosis* eine Thymin-Base zu finden ist, während bei anderen Mitgliedern des *M. tuberculosis*-Komplexes (insbesondere *M. africanum, M. microti, M. bovis* und *M. bovis BCG*) eine Cytosin-Base vorliegt (vgl. Fig. 1). Dieser Ein-Basen-Polymorphismus ist spezifisch für *M. tuberculosis* und ist vor allem stabil, wie eine vergleichende Untersuchung an über 100 *M. tuberculosis*-Stämmen weltweit bestätigte. Es wurde außerdem unerwarteterweise festgestellt, dass dieser eine Nukleotidpolymorphismus für die unterschiedlichen Reaktion im Nitratreduktasetest von *M. tuberculosis* im Vergleich zu den anderen Mitgliedern des *M. tuberculosis*-Komplexes verantwortlich ist (s. Beispiel 4).

Erfindungsgemäß bevorzugt wird zur Amplifikation des DNA-Fragments des *narGHJI-*Operons, mindestens ein Primer, insbesondere mindestens ein Primerpaar, eingesetzt, wobei die Primer die Nucleotidsequenz dargestellt in SEQ ID NO: 2 und/oder die Nucleotidsequenz dargestellt in SEQ ID NO: 3 enthalten, d.h. diese umfassen, insbesondere aber daraus bestehen, d.h. diese aufweisen. Erfindungsgemäß bevorzugt enthält das zur spezifischen Hybridisierung des amplifizierten DNA-Fragments bevorzugt eingesetzte mindestens eine Paar markierter Hybridisierungssonden Nucleotidsequenzen, ausgewählt aus der Gruppe der Nucleotidsequenzenpaare der Nucleotidsequenzen dargestellt in SEQ ID NO: 4 und in SEQ ID NO: 5, der Nücleotidsequenzen dargestellt in SEQ ID NO: 4 und in SEQ ID NO: 6, insbesondere besteht es daraus.

Der eindeutige Nachweis einer tuberkulösen Infektion durch Erregerstämme der Art *M. tuberculosis,* das heißt insbesondere der Stämme *M. tuberculosis* H37v (ATCC 25618) und *M. tuberculosis* Erdmann (ATCC 35801) sowie weiterer klinischer Stämme, findet erfindungsgemäß bevorzugt durch Analyse der Schmelztemperaturen der spezifischen Hybridisierung eines markierten Hybridisierungssondenpaars mit den Nucleotidsequenzen SEQ ID NO: 4 und SEQ ID NO: 5 oder dem Paar der komplementären Sequenzen mit einer *narGHJI*-Promotorregion, spezifisch für *M. tuberculosis,* statt. *M. tuberculosis* zeichnet sich dabei durch Schmelztemperaturen von insbesondere weniger als 61°C, bevorzugt weniger als 59°C, besonders bevorzugt von etwa 57°C, aus. Dem gegenüber besitzen die Erregerstämme der von *M. tuberculosis* zu unterscheidenden Mykobakterienarten *M. africanum, M. microti, M. bovis* und *M. bovis BCG* beim Einsatz des markierten Hybridisierungssondenpaars SEQ ID NO: 4 / SEQ ID NO: 5 Schmelztemperaturen von insbesondere mehr als 59°C, bevorzugt von mehr als 61°C, besonders bevorzugt von etwa 63°C.

In Abhängigkeit von den aktuell herrschenden Hybridisierungsbedingungen, beispielsweise von der Pufferzusammensetzung, von der Ausführung der Sonden als markierte Sonden oder durch Modifizierung der Nucleotidsequenz der Sonden kann es zu (meist geringen) Änderungen der absoluten Schmelztemperaturen kommen; selbstverständlich stellen alle diejenigen Verfahren, bei denen derart geänderte Schmelztemperaturen auftreten, bevorzugte Ausführungsformen hier vorgestellter erfindungsgemäßer Verfahren dar.

In einer weiteren Variante findet der eindeutige Nachweis einer tuberkulösen Infektion durch Erregerstämme der Art *M. tuberculosis,* das heißt insbesondere der Stämme *M. tuberculosis* H37v (ATCC 25618) und *M. tuberculosis* Erdmann (ATCC 35801) sowie weiterer klinischer Stämme, erfindungsgemäß bevorzugt durch Analyse der Schmelztemperaturen der spezifischen Hybridisierung eines markierten Hybridisierungssondenpaars mit den Nucleotidsequenzen SEQ ID NO: 4 und SEQ ID NO: 6 oder dem Paar der komplementären Sequenzen mit einer *narGHJI*-Promotorregion spezifisch für *M. tuberculosis* statt (vgl. Tab. 1). Die Erregerstämme der Art *M. tuberculosis* zeichnen sich dabei durch Schmelztemperaturen von insbesondere mehr als 58°C, bevorzugt mehr als 60°C, besonders bevorzugt von etwa 62°C aus. Demgegenüber besitzen die Erregerstämme von *M. africanum, M. microti, M. bovis* und *M. bovis BCG* beim Einsatz des markierten Hybridisierungssondenpaars SEQ ID NO: 4 / SEQ ID NO: 6 Schmelztemperaturen von insbesondere weniger als 62°C, bevorzugt von weniger als 60°C, besonders bevorzugt von etwa 58°C.

Die erfindungsgemäße Nukleinsäureamplifikation kann beispielsweise durch Polymerase-Kettenreaktion (*polymerase chain reaction,* PCR), Nucleinsäuresequenz-basierte Amplifikation (*nucleic acid sequence based amplification,* NASBA), Strangverdrängunsamplifikation (*strand displacement amplification,* SDA) oder Ligase-*Kettenreaktion (ligase chain reaction,* LCR) erfolgen. Daneben kommen selbstverständlich beliebige weitere Nukleinsäureamplifikationsverfahren in Betracht.

Ferner wurde überraschenderweise festgestellt, dass bei der Verwendung von mindestens einem Primer, insbesondere eines Primerpaares, umfassend die Nucleotidsequenz dargestellt in SEQ ID NO: 2 und/oder umfassend die Nucleotidsequenz dargestellt in SEQ ID NO: 3, zur Amplifikation mindestens eines DNA-Fragments der Promotorregion des *narGHJI*-Nitratreduktase-Operons von Mykobakterien, die Amplifikation lediglich bei Erregerstämmen des *M. tuberculosis*-Komplexes erfolgt, das heißt zu einem Amplifikationsprodukt führt. Bei nicht-tuberkulösen Erregern oder nichtmykobakteriellen Erregern findet hingegen keine Amplifikation statt, das heißt, es wird kein entsprechendes Amplifikationsprodukt erhalten. Durch die Verwendung der erfindungsgemäßen Primer gelingt dabei besonders vorteilhaft die eindeutige Unterscheidung zwischen den Erregerstämmen *M. tuberculosis* und anderen Erregerstämmen des *M. tuberculosis*-Komplexes (s.o.) sowie gegenüber nicht-tuberkulösen Mycobakterien und nicht-Mykobakterien, beispielsweise Actinomyceten, Erregern der Gattungen *Bacillus, Staphylococcus, Listeria, Enterococcus* sowie *Proteus, E. coli, Salmonella, Shigella, Klebsiella* oder *Pseudomonas* oder pilzlichen Erreger.

In einer bevorzugten Ausführungsform der vorgenannten Verfahren wird die Amplifikation der DNA-Fragmente mittels Polymerase-Kettenreaktion (PCR) durchgeführt. In einer besonders bevorzugten Ausführungsform ist die PCR eine Echtzeit-PCR (Rapid-Cycle-PCR).

Der Nachweis des für *M. tuberculosis* spezifischen Polymorphismus kann erfindungsgemäß durch spezifische Hybridisierung von einer oder mehreren Sonden erfolgen.

Im Echtzeit-PCR Verfahren ist es möglich, die Vervielfachung der PCR-Produkte in Echtzeit Amplifikations-Zyklus für Amplifikations-Zyklus zu beobachten. Besonders bevorzugt wird die Amplifikation in einem LightCycler^{™}-System der Firma Roche Molecular Biochemicals durchgeführt, das eine Ausgestaltung der Echtzeit-PCR ist. Zu diesem Zweck werden insbesondere der PCR-Ausgangsmischung neben der Polymerase, den Nucleotiden, den Pufferlösungen und den Primern auch Hybridisierungssonden zugegeben, die spezifisch an die gewünschten PCR-Amplifikationsprodukte binden. Dabei werden insbesondere zwei sequenzspezifische Oligonucleotidsonden verwendet, die mit verschiedenen Farbstoffen markiert sind. Die Sequenzen der erfindungsgemäßen markierten Hybridisierungssonden-Paare sind so ausgewählt, dass sie auf eine Weise an die Zielsequenzen des amplifizierten DNA-Fragments hybridisieren, dass insbesondere das 3'-Ende der einen Sonde dicht bei dem 5'-Ende der anderen Sonde liegt, wodurch die beiden Farbstoffe in unmittelbare Nachbarschaft zueinander gebracht werden, vorzugsweise beträgt der Abstand zwischen den beiden Sonden zwischen 1 und 5 Nucleotide. Insbesondere kommt es zu einem Fluoreszenz-Resonanz-Energietransfer (FRET; vgl. z.B. Heid et al., Genome Res. 6 (1996) 986-994) zwischen den beiden Farbstoffen der Hybridisierungssonden und damit zu einer Verschiebung des Fluoreszenzspektrums, wobei das Maß an Fluoreszenz in diesem Wellenlängenbereich eine Funktion der Menge an detektierter DNA ist. Die Detektion kann beispielsweise nach dem Verfahren gemäß WO 00/58505 erfolgen.

Durch das FRET-System sind erfindungsgemäß auch quantitative Messungen der Menge amplifizierter DNA-Fragmente vorgesehen. Die erfindungsgemäßen ausgewählten Hybridisierungssonden können quantitativ, also stöchiometrisch, an die amplifizierten Fragmente binden. Dabei ist die quantitative Hybridisierung insbesondere abhängig von der Temperatur und dem Homologiegrad der verwendeten Oligonucleotidsonden mit der detektierten Sequenz auf dem amplifizierten DNA-Fragment.

In einer bevorzugten Ausführungsform wird die fluorimetrische Detektion spezifischer DNA-Sequenzen in den amplifizierten Fragmenten nach einer Amplifikation der Fragmente mittels herkömmlicher PCR durchgeführt. In einer besonders bevorzugten Ausführungsform wird die fluorimetrische Detektion in einer Echtzeit-PCR während der Amplifikationsreaktionen durchgeführt, wobei beispielsweise die Zunahme an produzierter DNA als Zunahme im Fluoreszenzsignal verfolgt werden kann.

In einer bevorzugten Ausführungsform des erfindungsgemäßen Verfahrens erfolgt die spezifische Detektion der amplifizierten DNA-Fragmente nach Beendigung der Amplifikationsreaktion, wobei nach Hybridisierung des Hybridisierungssondenpaars, bevorzugt eines FRET-Paars an die zu detektierenden Regionen die Temperatur im Rahmen einer Schmelzkurenanalyse verändert wird, bevorzugt kontinuierlich erhöht wird, und gleichzeitig die in Abhängigkeit von der Temperatur emittierte Fluoreszenz gemessen wird. Auf diese Weise wird eine Schmelztemperatur bestimmt, bei der die Hybridisierungssonden, insbesondere das eingesetzte FRET-Paar, gerade nicht mehr an die zu detektierende Region des amplifizierten DNA-Fragments hybridisieren. Der wesentliche Aspekt einer Schmelzkurvenanalyse besteht darin, dass es bei Auftreten von Fehlpaarungen zwischen dem eingesetzten Hybridisierungssondenpaar und der Zielregion auf dem amplifizierten DNA-Fragment zu einer Reduktion des gemessenen Schmelzpunktes kommt. Auf diese Weise werden erfindungsgemäß mit Hybridisierungssonden, insbesondere mit einem FRET-Paar, die Regionen von DNA-Fragmenten identifiziert, deren Sequenzen sich voneinander insbesondere nur geringfügig, durch eine oder wenige Punktmutationen, in der Nucleotidsequenz unterscheiden.

Im Zusammenhang mit der vorliegenden Erfindung wird unter den Begriffen "Abschnitt", "Region", "Fragment", "Zielregion" oder "flankierende Region" mindestens ein zusammenhängender Bereich auf einem linearen, strangförmigen Desoxy- oder Ribonucleinsäuremolekül, das heißt DNA oder RNA, verstanden, welcher, in der Anzahl der Nucleotide dieses Moleküls gemessen, in 5' zu 3'-Leserichtung stromabwärts und/oder stromaufwärts von einem bestimmten nummerierten Nucleotid des Moleküls, das heißt einer bestimmten Position, aus bevorzugt 200, 100, 50, 40, 30, 20,10, 5, 4, 3 oder 2 Nucleotiden des Moleküls besteht.

Ein erfindungsgemäßer amplifizierter DNA-Abschnitt hat, zusätzlich zu der Sequenz an die der Primer bindet, vorzugsweise eine Länge von mindestens 1 und höchstens 500 Nukleotiden, bevorzugt höchstens 300 und besonders bevorzugt höchstens 155 Nukleotiden. Einzelne Längenwerte, die innerhalb der vorgenannten Bereiche liegen, sind ausdrücklich eingeschlossen, d.h. 2, 3, 4, 5, ..., 497, 498 und 499. Der amplifizierte DNA-Abschnitt kann selbstverständlich auch länger sein, wobei kürzere Abschnitte in der Praxis bevorzugt sein dürften.

Gemäß einer Ausführungsform der Erfindung weist das für das Verfahren verwendete Primerpaar mindestens eine der Sequenzen mit der SEQ ID NO:2 und SEQ ID NO:3 auf.

In einer Ausführungsform der Erfindung erfolgt der Nachweis des für *M. tuberculosis* spezifischen Polymorphismus mit mindestens einem Paar markierter Hybridisierungssonden, wobei eine Sonde an ihrem 3' Ende und die andere-Sonde an ihrem 5'-Ende markiert ist und die Sonden spezifisch so an das Amplifikat binden, daß ein Fluoreszenz Resonanz Energie Transfer (FRET) ermöglicht wird (s.o.).

In einer bevorzugten Ausführungsform weist das Sondenpaar die Sequenzen mit der SEQ ID NO:4 und 5, oder die komplementären Sequenzen davon und/oder die Sequenzen mit der SEQ ID NO: 4 und 6, oder die komplementären Sequenzen davon auf.

Gegenstand der Erfindung ist ferner ein Verfahren zum gemeinsamen Nachweis von Erregern des *M. tuberculosis*-Komplexes in klinischem Material, wobei die Gegenwart eines Amplifikationsprodukts der *narGHJI*-Promotorregion insbesondere durch mindestens eine Hybridisierungsonde spezifisch für das amplifizierte DNA-Fragment, bevorzugt durch das oben beschriebene Verfahren, detektiert wird und so Erregerstämme des *M. tuberculosis*-Komplexes gegenüber nicht-tuberkulösen Erregern und/oder gegenüber nicht-Mykobakterien nachgeweisen werden. In einer alternativen Variante wird das Auftreten eines Amplifikationsprodukts in an sich bekannter Weise, insbesondere durch elektrophoretische Methoden detektiert. In diesem Zusammenhang kann das Verfahren der vorliegenden Erfindung Teil eines umfassenderen Nachweisverfahrens sein, bei dem weitere, vorzugsweise erregerspezifische (Art- oder Gattungsspezifische) Nachweise durch Nukleinsäureamplifikation entsprechend spezifischer Sequenzabschnitte in parallelen Ansätzen oder in demselben Reaktions-Ansatz, insbesondere im Zusammenhang mit einer Multiplex-PCR, durchgeführt werden.

Ein wesentlicher Vorteil des erfindungsgemäßen Verfahrens ist, dass in einem Nachweisverfahren zur Diagnose von Mykobakterieninfektionen sowohl eine bestehende Infektion mit *M. tuberculosis* gegenüber anderen mikrobiellen Infektionen, eine Infektion mit *M. tuberculosis* gegenüber nicht-tuberkulösen Infektionen, und eine Infektion mit *M. tuberculosis* gegenüber anderen Mitgliedern des *M. tuberculosis*-Komplexes eindeutig, sicher und insbesondere gleichzeitig, bevorzugt in einem einzigen routinediagnostischen Ansatz, erkannt werden kann. Ein weiterer wesentlicher Vorteil des erfindungsgemäßen Verfahrens ist, dass zusätzlich, insbesondere gleichzeitig und eindeutig der Mykobakterienstamm der Art *M. tuberculosis* nachgewiesen und diese Art einzeln identifiziert werden kann. Die erfindungsgemäßen Nachweisverfahren erlauben daher insbesondere eine eindeutige und sichere Früherkennung einer Tuberkulose und die eindeutige und sichere Früherkennung der Art-Zugehörigkeit isolierter Erregerstämme des *M. tuberculosis*-Komplexes zu *M. tuberculosis.* Dies ermöglicht besonders vorteilhaft eine schnelle und gezielte Therapie des infizierten Organismus.

Das erfindungsgemäße Verfahren wird im Wesentlichen in der Routinediagnostik eingesetzt und im Wesentlichen in Routinelabors durchgeführt. Die Durchführung des Verfahrens in speziell ausgestatteten und aufwändig gestalteten Sicherheitslabors für die Kultivierung hochinfektiöser Erregerstämme ist nicht notwendig. Daher kann das erfindungsgemäße Verfahren einer breiten Anwenderschaft zugeführt werden.

Im Zusammenhang mit der vorliegenden Erfindung werden unter klinischem Material im Wesentlichen klinische Proben, das heißt Patientenmaterial wie Sputum, Bronchiallavage, Magensaft, Urin, Stuhl, Liquor, Knochenmark, Blut, aber auch Biopsien, insbesondere Punktat-Biopsien, beispielsweise aus Halslymphknoten, verstanden. Unter klinischem Material werden aber auch Kulturisolate, beispielsweise aus Flüssigkultur verstanden, insbesondere aus Flüssigkultur zur selektiven Kultivierung säurefester Stäbchen insbesondere aus Patientenmaterial.

Bevorzugt wird die mikrobielle DNA aus dem klinischen Material in an sich bekannter Weise, insbesondere mittels DNA-Präparationskits wie dem QIAmp™ DNA Mini Kit der Firma Qiagen, extrahiert.

Erfindungsgemäß bevorzugt wird die Probe aus der Gruppe klinischer Proben bestehend aus Sputum, Bronchiallavage, Magensaft, Urin, Stuhl, Liquor, Knochenmark, Blut und Biopsien ausgewählt.

Die vorliegende Erfindung betrifft ferner die zur Durchführung der oben angeführten Verfahren benötigten Primer, Primerpaare, Sonden und Sondenpaare.

Erfindungsgemäß eingesetzt wird ein Primerpaar, das zur Amplifikation eines DNA-Abschnitts aus der in SEQ ID NO: 1 gezeigten Sequenz, die einen Abschnitt aus dem Bereich des *narGHJI*-Nitratreduktase-Operons umfasst, geeignet ist, wobei der DNA-Abschnitt die Position -215 in 5' zu 3' Leserichtung strangaufwärts des Translationsstartcodons GTG des *narGHJI*-Nitratreduktase-Operons umfaßt.

Erfindungsgemäß bevorzugt wird zur Amplifikation eines DNA-Fragments mit einem DNA-Polymorphismus des *narGHJI*-Gens spezifisch für Erregerstämme der Art *M. tuberculosis* aus der extrahierten DNA mit einer Länge von 155 bp das Primerpaar verwendet, bei dem der Vorwärtsprimer die Nucleotidsequenzen gemäß SEQ ID NO: 2 und der Rückwärtsprimer die Nucleotidsequenz gemäß SEQ ID NO: 3 umfasst bzw. aufweist. Ein weiterer Gegenstand der Erfindung ist daher auch mindestens ein Oligonucleotid-Primer, bevorzugt mindestens ein Oligonucleotid-Primerpaar zur Amplifikation eines DNA-Fragments der *M. tuberculosis*-spezifischen Promotorregion des *narGHJI-*Nitratreduktase-Gens, enthaltend, insbesondere bestehend aus, Nucleinsäuremolekülen mit Nucleotidsequenzen dargestellt in SEQ ID NO: 2 und/oder SEQ ID NO: 3 bzw. diese Nucleotidsequenzen umfassend.

In einer bevorzugten Ausführungsform weist mindestens ein Primer eines Primerpaares die in SEQ ID NO: 2 angegebene Sequenz auf oder umfasst dieselbe.

In einer weiteren bevorzugten Ausführungsform weist mindestens ein Primer eines Primerpaares die in SEQ ID NO: 3 angegebene Sequenz auf oder umfasst dieselbe.

In einer besonders bevorzugten Ausführungsform weisen die Primer eines Primerpaares die in SEQ ID NO: 2 und die in SEQ ID NO: 3 angegebenen Sequenzen auf.

Erfindungsgemäße Primer können die in SEQ ID NO: 2 angegebene Sequenz oder die in SEQ ID NO: 3 angegebene Sequenz aufweisen.

Eine Ausführungsform der Erfindung betrifft die Verwendung eines der oben genannten Primer oder Primerpaares zum spezifischen Nachweis von *M. tuberculosis,* d.h. durch Amplifikation von in einer biologischen Probe enthaltene *M. tuberculosis*-DNA und spezifischen Nachweis des erhaltenen Amplifikats (s.o.).

Die Erfindung betrifft bevorzugt auch Primer und/oder Primerpaare zur Amplifikation der erfindungsgemäß eingesetzten DNA-Fragmente, welche gegenüber den vorgenannten erfindungsgemäßen Primem umfassend die Nucleotidsequenzen dargestellt in SEQ ID NO: 2 und 3 jeweils degenerierte, mutierte oder modifizierte Sequenzen oder Fragmente davon, die mit der jeweiligen Nucleotidsequenz dargestellt in SEQ ID NO: 2 und 3, wovon sie jeweils abgeleitet sind, hybridisieren, wobei jeweils ein Homologiegrad, bevorzugt über die gesamte Länge der Sequenz, von mindestens 92 %, bevorzugt von mindestens 97%, besonders bevorzugt von mindestens 98 % zu der ursprünglichen Nucleotidsequenz, besteht. Dabei weisen die abgeleiteten Fragmente jeweils eine Sequenzlänge auf, welche bevorzugt maximal etwa 98% der Länge der Nucleotidsequenz, besonders bevorzugt maximal etwa 95%, maximal etwa 90%, maximal etwa 75%, maximal etwa 50% oder maximal etwa 25% beträgt. Insbesondere bevorzugt ist das abgeleitete Fragment um maximal 10, um maximal 5, um 4, 3, oder 2 Nucleotide oder um ein Nucleotid gegenüber der ursprünglichen Nucleotidsequenz verkürzt.

Im Zusammenhang mit der vorliegenden Erfindung wird unter dem Begriff "modifizierte Sequenz" oder "modifizierte Nucleotidsequenz" eine Nucleinsäuresequenz verstanden, die sich durch Austausch, Inversion, Deletion oder Addition von mindestens einem Nucleotid, auch einem ungewöhnlichen oder synthetischen Nucleotid, von ihrer ursprünglichen Sequenz, in mindestens einem Nucleotid, bevorzugt in zwei Nucleotiden, unterscheidet. In diesem Zusammenhang wird unter dem Begriff "modifiziert" eine Eigenschaft verstanden, die sich auf eine modifizierte Nucleotidsequenz bezieht.

Im Zusammenhang mit der vorliegenden Erfindung wird unter den Formulierungen "Primer, welcher die Nucleotidsequenz umfasst" oder "Hybridisierungssonde, welche die Nucleotidsequenz umfasst" oder ähnlichen verstanden, dass die jeweiligen Primer und Sonden die Nucleotidsequenzen aufweisen, das heißt, aus den konkret genannten Nucleotidsequenzen allein bestehen. Unter der Formulierung wird auch verstanden, dass die jeweiligen Primer und Sonden neben den konkret genannten Nucleotidsequenzen gegebenenfalls aus mindestens einer weiteren zusätzlichen Sequenz bestehen. Diese zusätzliche Sequenz flankiert die konkret genannten Nucleotidsequenzen und besitzt eine Sequenzlänge, welche bevorzugt maximal etwa 100% der Länge der konkret genannten Nucleotidsequenz, besonders bevorzugt maximal etwa 75%, maximal etwa 50%, maximal etwa 25%, maximal etwa 10%, maximal etwa 5% oder maximal etwa 2% beträgt. Insbesondere bevorzugt besitzt die zusätzliche Sequenz eine Länge von 10 bis 5 Nucleotiden, von 4, 3, 2 Nucleotiden oder besteht aus einem einzigen Nucleotid.

Eine erfindungsgemäße Hybridisierungssonde ist eine Sonde, die zum spezifischen Nachweis durch spezifische Hybridisierung des für *M. tuberculosis* spezifischen Polymorphismus, der an Position -215 in 5' zu 3' Leserichtung strangaufwärts des Translationsstartcodons GTG des *narGHJI-*Nitratreduktase-Operons lokalisiert ist, geeignet ist.

Ein erfindungsgemäßes Hybridisierungssonden-Paar ist ein Sonden-Paar, das zum spezifischen Nachweis durch spezifische Hybridisierung des für *M. tuberculosis* spezifischen Polymorphismus, der an Position -215 in 5' zu 3' Leserichtung strangaufwärts des Translationsstartcodons GTG des *narGHJI*-Nitratreduktase-Operons lokalisiert ist, geeignet ist.

Ein weiterer Gegenstand der Erfindung ist auch mindestens eine Oligonucleotid-Hybridisierungssonde, welche spezifisch mit einer *M. tuberculosis*-spezifischen Promotorregion des *narGHJI*-Nitratreduktase-Operons hybridisiert, welche das Nucleinsäuremolekül mit der Nucleotidsequenz dargestellt in SEQ ID NO: 5 oder mit der komplementären Sequenz davon enthält, insbesondere daraus besteht. Ein weiterer Gegenstand der Erfindung ist auch mindestens ein Oligonucleotid-Hybridisierungssondenpaar, welches die Nucleinsäuremoleküle mit den Nucleotidsequenzen dargestellt in SEQ ID NO: 4 und in SEQ ID NO: 5 oder mit den komplementären Sequenzen davon enthält, insbesondere daraus besteht.

Ein weiterer Gegenstand der Erfindung ist auch eine weitere oder alternative Oligonucleotid-Hybridisierungssonde, welche spezifisch mit einer *M. tuberculosis-*spezifischen Promotorregion des *narGHJI*-Nitratreduktase-Operons hybridisiert, welche das Nucleinsäuremolekül mit der Nucleotidsequenz dargestellt in SEQ ID NO: 6 oder mit der komplementären Sequenz davon enthält, insbesondere daraus besteht. Ein weiterer Gegenstand der Erfindung ist auch mindestens ein Oligonucleotid-Hybridisierungssondenpaar, welches die Nucleinsäuremoleküle mit den Nucleotidsequenzen dargestellt in SEQ ID NO: 4 und in SEQ ID NO: 6 oder mit den komplementären Sequenzen davon enthält, insbesondere daraus besteht.

In einer bevorzugten Ausführungsform weist mindestens eine Sonde eines Hybridisierungssonden-Paares die in SEQ ID NO: 4 angegebene Sequenz oder die komplementäre Sequenz davon, oder die in SEQ ID NO: 5 angegebene Sequenz oder die komplementäre Sequenz davon, oder die in SEQ ID NO: 6 angegebene Sequenz oder die komplementäre Sequenz davon auf.

In einer besonders bevorzugten Ausführungsform weisen die Sonden eines Hybridisierungssonden-Paares die in Sequenz ID NO: 4 und SEQ ID NO: 5 angegebenen Sequenzen oder die komplementären Sequenzen davon, oder die in Sequenz ID NO: 4 und SEQ ID NO: 6 angegebenen Sequenzen oder die komplementären Sequenzen davon auf.

Erfindungsgemäße Hybridisierungssonden können die in SEQ ID NO: 4 angegebene Sequenz oder die komplementäre Sequenz davon, oder die in SEQ ID NO: 5 angegebene Sequenz oder die komplementäre Sequenz davon, oder die in SEQ ID NO: 6 angegebene Sequenz oder die komplementäre Sequenz davon aufweisen.

Erfindungsgemäß bevorzugt wird die Detektion der DNA-Fragmente mit den vorgenannten Hybridisierungssonden durchgeführt, welche als FRET-Paare ausgeführt, d.h. markiert, sind, wobei jeweils ein Hybridisierungssonden-Partner als Donor-Komponente = "Anker-Sonde" ("anchor probe") ausgeführt ist, die vorzugsweise am 3'-terminalen Nucleotid mit einem Farbstoff, vorzugsweise mit einem Fluoreszenz-Farbstoff, besonders bevorzugt mit Fluorescein assoziiert ist. Erfindungsgemäß sind bevorzugt als Anker-Sonden vorgesehen die Hybridisierungssonden umfassend die Nucleotidsequenz dargestellt in SEQ ID NO: 4 oder die komplementäre Sequenz davon.

Der jeweils andere Hybridisierungssonden-Partner, des Hybridisierungssonden-Paars ist als Akzeptor-Komponente = Sensor-Sonde ("sensor probe") ausgeführt, welche vorzugsweise am 5'-terminalen Nucleotid mit einem weiteren Farbstoff, vorzugsweise mit einem Rhodamin-Derivat assoziiert ist. Erfindungsgemäß sind bevorzugt als Sensor-Sonden vorgesehen die Hybridisierungssonden umfassend die Nucleotidsequenzen dargestellt in SEQ ID NO: 5, 6 oder die jeweils komplementären Sequenzen davon. In bevorzugten Varianten der vorgenannten Ausführungsformen ist das Rhodamin-Derivat LightCycler-Red 640^{®}; in weiteren bevorzugten Varianten der vorgenannten Ausführungsform ist das Rhodamin-Derivat LightCycler-Red 705^{®}; in weiteren bevorzugten Varianten der vorgenannten Ausführungsform ist das Rhodamin-Derivat Cy5. Besonders bevorzugt tragen die für den erfindungsgemäßen artspezifischen Nachweis von *M. tuberculosis* eingesetzten markierten Sensor-Sonden den Farbstoff LightCycler-Red 640^{®} oder LightCycler-Red 705^{®}.

Eine weitere Ausführungsform der Erfindung betrifft die Verwendung einer der oben beschriebenen Hybridisierungssonden oder eines der oben beschriebenen Hybridisierungssondenpaare zum spezifischen Nachweis von *M. tuberculosis.*

Die vorliegende Erfindung umfaßt selbstverständlich auch Mittel, wie z.B. Kits (Vorrichtungen), zur Durchführung eines erfindungsgemäßen Verfahrens.

Ein Kit umfaßt mindestens ein Primerpaar, das zur Amplifikation eines DNA-Abschnitts aus der in SEQ ID NO: 1 gezeigten Sequenz, die einen Abschnitt aus dem Bereich des *narGHJI*-Nitratreduktase-Operons umfasst, geeignet ist, wobei der DNA-Abschnitt die Position -215 in 5' zu 3' Leserichtung strangaufwärts des Translationsstartcodons GTG des *narGHJI*-Nitratreduktase-Operons umfaßt, und mindestens eine Hybridisierungssonde oder ein Hybridisierungssonden-Paar, die bzw. das zum spezifischen Nachweis des für *M. tuberculosis* spezifischen Polymorphismus, der an Position -215 in 5' zu 3' Leserichtung strangaufwärts des Translationsstartcodons GTG des *narGHJI*-Nitratreduktase-Operons lokalisiert ist, geeignet ist, dabei umfaßt das Kit sowohl ein Primerpaar, bei dem die Primer die in SEQ ID NO: 2 und SEQ ID NO: 3 angegebenen Sequenzen aufweisen, als auch ein Hybridierungssonden-Paar, bei dem die Sonden die in SEQ ID NO: 4 und SEQ ID NO: 5 oder die komplementären Sequenzen davon, oder aber die in SEQ ID NO: 4 und SEQ ID NO: 6 oder die komplementären Sequenzen davon aufweisen.

In einer weiteren Ausführungsform umfaßt das Kit zusätzlich weitere, für die Durchführung einer Nukleinsäureamplifikation und/oder Nachweisreaktion des Amplifikats erforderliche oder nützliche Geräte, Reagenzien und/oder Hilfsmittel,

Ein weiterer Gegenstand der Erfindung ist auch die Verwendung mindestens eines *M. tuberculosis*-spezifischen DNA-Polymorphismus in der Promotorregion des *narGHJI-*Nitratreduktase-Operons von Mykobakterien, insbesondere an Position -215 in 5' zu 3'-Leserichtung strangaufwärts des Translationsstartcodons GTG des *narGHJI*-Operons, zum spezifischen Nachweis einer Infektion mit *M. tuberculosis,* dabei ist dieser Genomabschnitt dadurch gekennzeichnet, dass er die Nucleinsäuresequenz dargestellt in SEQ ID NO: 6 oder die komplementäre Sequenz davon enthält, insbesondere aus dieser besteht. Bevorzugt erfolgt die erfindungsgemäße Verwendung in mindestens einem PCR-Ansatz mit anschließender oder gleichzeitiger Hybridisierung, beispielsweise in einer Echtzeit-PCR oder in an sich bekannten Verfahren der klassichen Hybridisierung, der DNA-Sequenzierung, insbesondere in einem Kapillarsequenziergerät, der allelspezifischen PCR, des OLA ("oligonucleotideligation assay"), des SSCP ("single strand conformation polymorphism") oder der denaturierenden Gradienten-Gelektrophorese (DGGE). Alternative Verfahren zur Amplifikation bestehen dabei neben der PCR beispielsweise in den bekannten Verfahren NASBA, SDA oder LCR.

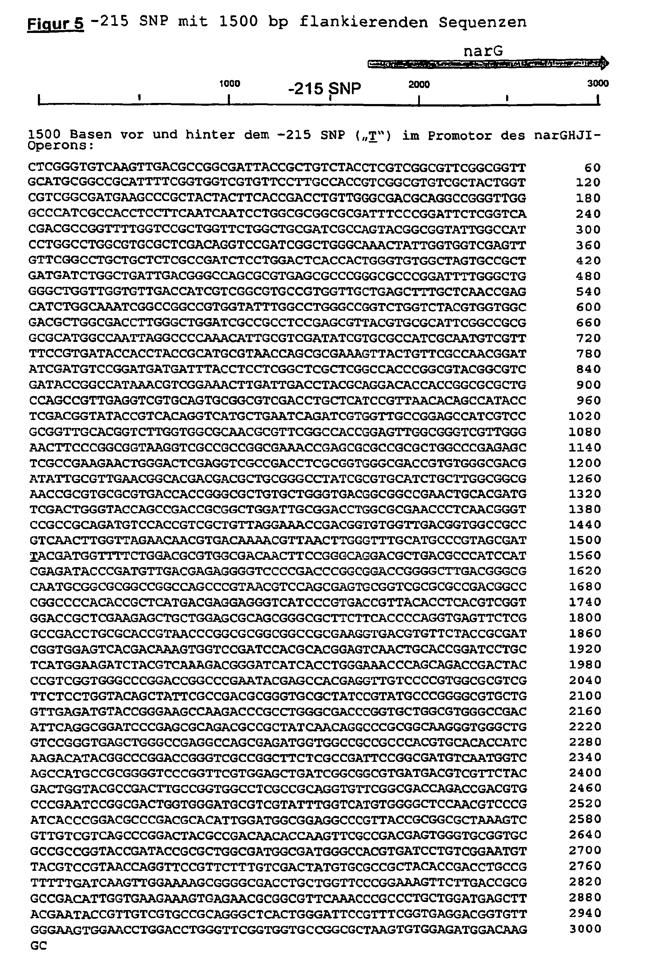

Die Erfindung wird anhand des anhängenden Sequenzprotokolls, das die Sequenzen Nr. 1 bis 6 enthält, anhand der Figuren 1 bis 5 sowie anhand der Beispiele 1 bis 4 näher erläutert.

SEQ ID NO: 1 - Region im Bereich des *narGHJI*-Operons, die den für *M. tuberculosis* spezifischen Polymorphismus an Position -215 im *nar*GHJI Promotor umfaßt;

SEQ ID NO: 2 - Vorwärtsprimer zur Amplifikation eines 155 bp-Fragments des *narGHJI*-Promotors von Mykobakterien enthaltend einen DNA-Polymorphismus spezifisch für *M. tuberculosis,*

SEQ ID NO: 3 - Rückwärtsprimer zu SEQ ID NO: 2;

SEQ ID NO: 4 - Hybridisierungssonde (antisense), insbesondere Anker-Sonde und Donor-Komponente eines Sondenpaars zur Detektion der artspezifischen Region des *narGHJI*-Promotors von Mykobakterien;

SEQ ID NO: 5 - Hybridisierungssonde (antisense), insbesondere Sensor-Sonde und Akzeptor-Komponente eines Sondenpaars zur Detektion der artspezifischen Region des *narGHJI*-Promotors von Mykobakterien;

SEQ ID NO: 6 - Hybridisierungssonde (antisense), insbesondere Sensor-Sonde und Akzeptor-Komponente eines Sondenpaars zur Detektion der artspezifischen Region des *narGHJI*-Promotors von Mykobakterien.

### Beschreibung der Figuren:

Figur 1 - zeigt ein Alignment der *narG* Promotor-Regionen von *M*. *tuberculosis, M*. *bovis* und *M.bovis* BCG. Der markierte Bereich zeigt den für *M*. *tuberculosis* spezifischen Nukeotidpolymorphismus ("T").
Figur 2 - zeigt Schmelzkurven der Hybridisierung des spezifischen Hybridisierungssondenpaars mit SEQ ID NO: 4 /SEQ ID NO: 5 mit der *M*. tuberculosis-spezifischen Region im amplifizierten 155 bp-Fragment des narGHJ-Promotors.
Figur 3 - zeigt Schmelzkurven der Hybridisierung des spezifischen Hybridisierungssondenpaars mit SEQ ID NO: 4 / SEQ ID NO: 6 mit der *M*. *tuberculosis*-spezfischen Region im amplifizierten 155 bp-Fragment des *narGHJ*-Promotors.
Figur 4 - zeigt einen diagnostischen Nitratreduktasetest bei eigens hergestellten T-215C-Mutanten von *M*. *tuberculosis,* bei den zugehörigen Wildtypen sowie bei *M*. *bovis* und *M*. *bovis* BCG. Die Rotfärbung (dunkel) durch den aus dem Naphtylamid und Sulfanilsäure gebildeten Diazoniumfarbstoff zeigt die Nitritakkumulation im Medium an: Allein die Wildtypen von *M*. *tuberculosis* weisen eine Nitritakkumulation auf; die T-215C-Mutanten davon besitzen (wieder) den M. *bovis* -Phänotyp.
Figur 5 - zeigt einen Bereich des *narG* Gens (SEQ ID NO: 1), der den für *M*. *tuberculosis* spezifischen Nukleotidpolymorphismus (**T**) an Position -215 strangaufwärts des Translationsstartcodons GTG aufweist.

### BEISPIELE

### Beispiel 1: DNA-Isolierung

### a) aus klinischem Material

Mikrobielle DNA wird aus klinischen Proben bestehend aus Sputum, Bronchiallavage, Magensaft, Urin, Stuhl, Liquor, Knochenmark, Blut oder Punktat-Biopsien in an sich bekannter Weise zum Beispiel mittels eines QIAmp™ DNA Mini Kit (Firma Qiagen, Hilden, Deutschland) aufgereinigt, das heißt extrahiert.

### b) aus Kulturisolaten

Kulturen zu diagnostizierender Mikroorganismen aus Patientenproben werden in einem automatisierten Zellkultursystem BACTEC™ MGIT™ (Becton, Dickinson and Company Diagnostic Systems, USA) in Flüssigkulturen unter Bedingungen, welche die Kultivierung säurefester Stäbchen, insbesondere von Mykobakterien, unterstützen, angezüchtet. Aus den positiven Kulturen wird die mikrobielle DNA beispielsweise mittels mechanischer Disruption erhalten. Die mikrobielle DNA wird in an sich bekannter Weise zum Beispiel mittels eines QIAmp™ DNA Mini Kit (Qiagen, Hilden, Deutschland) aus den Kulturisolaten extrahiert und gegenbenenfalls aliquotiert.

### Beispiel 2: PCR-Amplifikation

Zur Amplifikation in einer optimierten LightCycler™-PCR wird ein Ansatz beinhaltend die fertig erhältliche Mischung "LightCycler FastStart DNA Master Hybridisation Probes" (Katalog-Nr. 239272, Firma Roche Molecular Biochemicals) gewählt.

Es wird folgende Reaktionsmischung für die LightCycler-Reaktion hergestellt:
- FastStart™ *Taq* Polymerase
- Reaktionspuffer
- Desoxi-Nucleosid-Triphosphat-Mischung (dNTP)
- 3 mmol/l MgCl₂ (Endkonzentration)
- Primer, je Primer: 18 pmol, entsprechend 1,1 µmol/l Endkonzentration
- Oligonucleotid-FRET-Sonden-Paar
   je Sonde: 2 pmol, entsprechend 100 nmol/l Endkonzentration

Diese Reaktionsmischung wird durch Puls-Zentrifugation in die Glaskapillaren des LightCycler-Systems verbracht und die Amplifikation nach dem "Hot Start"-Prinzip nach einer anfänglichen Denaturierung bei 95°C für 10 Minuten mit den folgenden Schritten durchgeführt:
1. Denaturierung bei 95°C für 3 Sekunden
2. Primer-Hybridisierung bei Temperaturen von 68°C bis 62°C für 2 Sekunden ("touch-down-annealing")
3. Polymerisation bei 72°C für 40 Sekunden

Die Schritte 1 bis 3 werden insgesamt 50 mal zyklisch abgearbeitet, wobei für die ersten 5 Zyklen die Hybridisierung in Schritt 2 bei 68°C erfolgt und bei den nachfolgenden 6 Zyklen die Temperatur auf 62°C in Schritten von 1 °C pro Zyklus reduziert wird und für die restlichen Zyklen bei 62°C durchgeführt wird. Die Rate der Temperaturänderung liegt bei allen Schritten bei 20°C pro Sekunde.

Zur Amplifikation der den *M*. *tuberculosis*-spezifischen DNA-Polymorphismus enthaltenden Region des *narGHJI*-Promotors wird der Primer mit der Nucleotidsequenz SEQ ID NO: 2 als Vorwärtsprimer und der Primer mit der Nucleotidsequenz SEQ ID NO: 3 als Rückwärtsprimer eingesetzt. Es werden 155 bp-Fragmente des *narGHJI*-Promotors amplifiziert. Es zeigt sich weiter, dass die Verwendung dieser Primer lediglich bei Erregerstämmen des *M*. *tuberculosis*-Komplexes zu einem Amplifikationsprodukt führen, nicht aber bei nicht-tuberkulösen Erregern oder nicht-myobakteriellen Erregern. Daher lässt die Verwendung der Primer mit den Nucleotidsequenzen SEQ ID NO: 2 und/oder SEQ ID NO: 3 auch die eindeutige Unterscheidung von Erregern von Arten des *M*. *tu*berculosis-Komplexes gegenüber Erregern von nicht-tuberkulösen Arten und gegenüber Erregern von nicht-Mykobakterien zu.

### Beispiel 3: Detektion und Schmelzkurvenanalyse

Zur Detektion der amplifizierten Fragmente werden in die Reaktionsmischung (siehe Beispiel 2) eingesetzte FRET-markierte Hybridisierungssondenpaare verwendet, wobei jeweils ein Hybridisierungssonden-Partner (SEQ ID NO: 4) als Donor-Komponente am 3'-terminalen Nucleotid mit Fluorescein assoziiert ist, und der jeweils andere Hybridisierungssonden-Partner (SEQ ID NO: 5 oder 6 als Akzeptor-Komponente am 5'-terminalen Nucleotid mit LightCycler-Red™ 640 assoziiert ist. Die bei der Detektion mit den Hybridisierungssonden unmittelbar nach dem letzten Amplifikationszyklus erfolgende Schmelzkurvenanalyse beginnt mit der Denaturierung der amplifizierten Fragmente bei 95°C für 30 Sekunden, worauf eine Hybridisierung mit den vorgenannten FRET-Paaren bei 38°C für 30 Sekunden folgt. Zur Bestimmung der Schmelzkurve der Hybridisierung wird die Temperatur anschließend kontinuierlich von 38°C auf 80°C mit einer Rate von 0,2°C pro Sekunde erhöht, wobei die von den konjugierten FRET-Paaren emittierte Fluoreszenz kontinuierlich aufgezeichnet wird. Die Fluoreszenz erlischt regelmäßig sobald mindestens ein Hybridisierungssonden-Partner abschmilzt. Zur Auswertung des Fluoreszenzsignals wird das LightCycler-"Run Profile"-Programm in der Version 3.5.3 eingesetzt, wobei die Verstärkung des F2- und F3-Kanals des photometrischen Detektors des LightCycler-Systems automatisch eingestellt wird.

Zur spezifischen Hybridisierung der den *M*. *tuberculosis*-spezifischen DNA-Polymorphismus enthaltenden Region des *narGHJI*-Promotors wird das FRET-markierte Hybridisierungssondenpaar mit den Nucleotidsequenzen SEQ ID NO: 4 / SEQ ID NO: 5 eingesetzt. Alternativ wird zur spezifischen Hybridisierung der den *M*. tuberculosis-spezifischen DNA-Polymorphismus enthaltenden Region des *narGHJI-*Promotors das FRET-markierte Hybridisierungssondenpaar mit den Nucleotidsequenzen SEQ ID NO: 4 / SEQ ID NO: 6 eingesetzt.

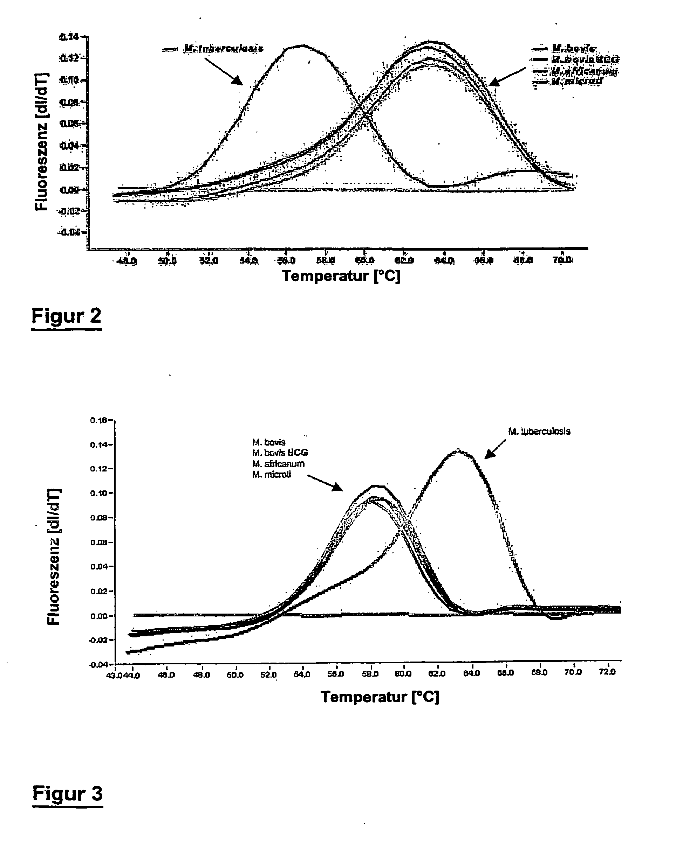

Die Figuren 2 und 3 und die Tabelle 1 zeigen die Ergebnisse der Schmelzkurvenanalyse.

### Beispiel 4: Vergleich des erfindungsgemäßen Verfahrens mit dem herkömmlichen Nitratreduktasetest.

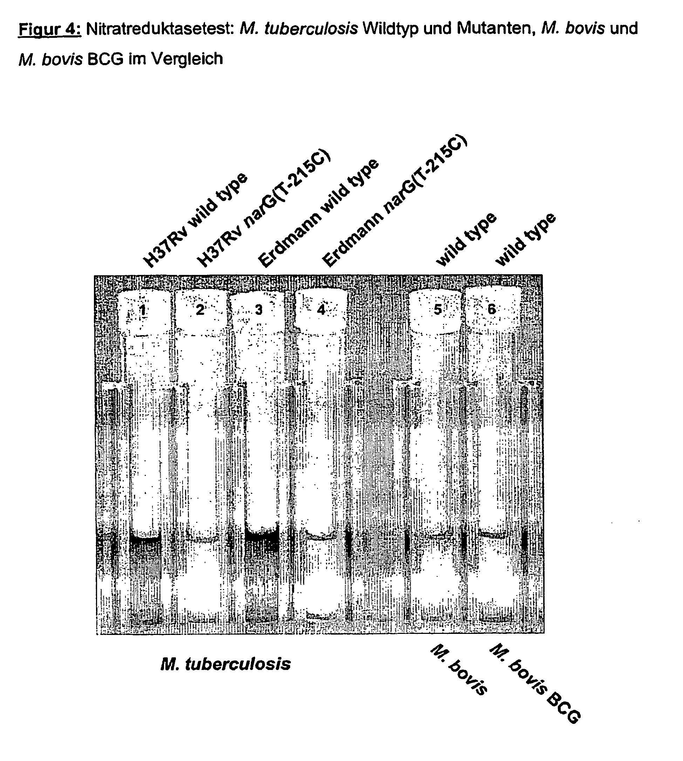

In Vergleichsversuchen wurden verschiedene, *M*. *tuberculosis, M*. *bovis* und *M*. *bovis* BCG enthaltende Proben dem erfindungsgemäßen Verfahren sowie dem herkömmlichen Nitratreduktasetest (vgl. Figur 4) unterzogen.

Es wurden folgende Proben eingesetzt, wobei die Probennummem der Nummerierung in Figur 4 entsprechen:
*M*. *tuberculosis:*
   1. H37Rv Wildtyp;
   2. H37Rv *nar*G (T-215C);
   3. Erdmann Wildtyp;
   4. Erdmann *nar*G (T-215C);
*M. bovis:*
   5. Wildtyp
*M. bovis* BCG:
   6. Wildtyp

Die Proben Nr. 1 und 3 wurden unter Verwendung des erfindungsgemäßen Verfahrens (vgl. Beispiele 3 und 4) positiv getestet, d.h. *M*. *tuberculosis* wurde spezifisch nachgewiesen, wobei eine eindeutige Unterscheidung von den Mycobakterienstämmen Nr. 5 und 6 möglich war. Die Proben 2 und 4, bei denen die für T an Position -215 kodierende Sequenz gentechnisch so modifiziert ist, dass sie für C kodiert, zeigten in der Schmelzkurve niedrigere und mit den *M*. *bovis* und *M*. *bovis* BCG enthaltenden Proben 5 und 6 vergleichbare Werte.

Der biochemische Test, Nitratreduktasetest, bestätigte das Ergebnis, wobei die unmodifizierten *M*. *tuberculosis*-Proben Nr. 1 und 3 positiv getestet wurden, während bei den *M. bovis* und *M. bovis* BCG enthaltenden Proben sowie den Proben, die gentechnisch an Position -215 durch T->C Austausch modifizierte *M*. *tuberculosis*-Stämme enthielten, keine Farbreaktion beobachtet wurde, was die Signifikanz des Polymorphismus an Position -215 für das erfindungsgemäße Nachweisverfahren bestätigt.

**Tabelle 1**

| Art | Stamm | Schmelztemperatur [°C] der Hybridisierungssonden spezifisch für | |
|---|---|---|---|
| | | *M*. *tuberculosis* | *M. tuberculosis* |
| | | Sonde: SEQ ID NO: 6 | Sonde: SEQ ID NO: 5 |
| | | Zielregion: *narGHJI-Pro.* | Zielregion: *narGHJI-Pro.* |
| *M. tuberculosis* | H37v ATCC 25618 | 62,3° | 56,8° |
| | Erdmann ATCC 35801 | 62,2° | 57,0° |
| | klinische Stämme (n = 33) | 62,2° (SD = 0,29) | 56,9° (SD = 0,36) |
| *M*. *africanum* | klinische Stämme (n = 3) | 57,9° (SD = 0,14) | 63,4° (SD = 0,21) |
| *M*. *microti* | klinische Stämme (n = 2) | 57,0° (SD = 0,14) | 63,2° (SD = 0,28) |
| *M*. *bovis* | ATCC 19210 | 58,2° | 63,1° |
| | klinische Stämme (n = 12) | 58,0° (SD = 0,22) | 63,2° (SD = 0,30) |
| *M*. *bovis* BCG | Pasteur ATCC 35734 | 57,9° | 63,6° |
| | Copenhagen ATCC 27290 | 58,0° | 63,5° |
| | Moreau ATCC 35736 | 58,2° | 63,7° |
| | Tice ATCC 35743 | 58,2° | 63,5° |
| | Connaught ATCC 35745 | 58,2° | 63,7° |
| | klinische Stämme (n = 4) | 57,9° (SD = 0,26) | 63,2° (SD =0,26) |

## Patentansprüche

1. Verfahren zum spezifischen Nachweis von *Mycobacterium tuberculosis (M. tuberculosis)* in klinischem Material, bei dem man
mikrobielle DNA aus dem klinischen Material extrahiert,
mindestens ein DNA-Fragment des Nitratreduktase-Operons von Mykobakterien, das heißt des narGHJI-Operons, welches Nitratreduktase codiert, aus der extrahierten DNA amplifiziert, wobei das amplifizierte DNA-Fragment oder eines der amplifizierten DNA-Fragmente eine Zielregion beinhaltet, welche mindestens einen für *M. tuberculosis* spezifischen DNA-Polymorphismus umfasst, der an Position - 215 in 5' zu 3' Leserichtung strangaufwärts des Translationsstartcodons GTG des narGHJI- Operons lokalisiert ist,
wobei die spezifische Hybridisierung des mindestens einen amplifizierten DNA-Fragments mit mindestens einer Hybridisierungssonde detektiert wird.

2. Verfahren nach Anspruch 1, **dadurch gekennzeichnet, dass** die Nukleinsäureamplifikation durch PCR, NASBA, SDA oder LCR erfolgt.

3. Verfahren nach Anspruch 2, **dadurch gekennzeichnet, dass** die PCR eine Echtzeit-PCR ist,

4. Verfahren nach den Ansprüchen 1 bis 3, **dadurch gekennzeichnet, dass** der Nachweis des für *M. tuberculosis* spezifischen Polymorphismus durch spezifische Hybridisierung von einer oder mehreren Sonden erfolgt.

5. Verfahren nach den Ansprüchen 1 bis 4, **dadurch gekennzeichnet, dass** das Primerpaar mindestens eine der Sequenzen mit der SEQ ID NO: 2 und SEQ ID NO:3 umfasst.

6. Verfahren nach den Ansprüchen 1 bis 4, **dadurch gekennzeichnet, dass** der amplifizierte DNA-Abschnitt eine Länge von 155 Nukleotiden umfasst und das Primerpaar die Sequenzen mit der SEQ ID NO: 2 und SEQ ID NO:3 umfasst.

7. Verfahren nach den Ansprüchen 1 bis 6, **dadurch gekennzeichnet, dass** der Nachweis des für *M. tuberculosis* spezifischen Polymorphismus mit markierten Hybridisierungssondenpaaren erfolgt, wobei eine Sonde an ihrem 3' Ende und die andere Sonde an ihrem 5'-Ende markiert ist und die Sonden spezifisch so an das Amplifikat binden, dass ein Fluoreszenz Resonanz Energie Transfer (FRET) ermöglicht wird.

8. Verfahren nach Anspruch 7, **dadurch gekennzeichnet, dass** das Sondenpaar die Sequenzen mit der SEQ ID NO: 4 und 5, oder die komplementären Sequenzen davon und/oder die Sequenzen mit der SEQ ID NO: 4 und 6, oder die komplementären Sequenzen davon umfasst.

9. Verfahren nach den Ansprüchen 1 bis 8, **dadurch gekennzeichnet, dass** das klinische Material ausgewählt ist aus der Gruppe klinischer Proben bestehend aus Sputum, Bronchiallavage, Magensaft, Urin, Stuhl, Liquor, Knochenmark, Blut, Biopsien und Kulturisolate.

10. Primerpaar, das zur Amplifikation eines DNA-Abschnitts, der einen Abschnitt aus dem Bereich des narGHJI-Nitratreduktase-Operons umfasst, geeignet ist, wobei der DNA-Abschnitt die Position -215 in 5' zu 3' Leserichtung strangaufwärts des Translationsstartcodons GTG des narGHJI-Nitratreduktase-Operons umfasst, **dadurch gekennzeichnet, dass** mindestens ein Primer die in SEQ ID NO: 2 oder SEQ ID NO: 3 angegebene Sequenz umfasst.

11. Primerpaar nach Anspruch 10, **dadurch gekennzeichnet, dass** die Primer die in SEQ ID NO: 2 und die in SEQ ID NO: 3 angegebenen Sequenzen umfassen.

12. Primer, **dadurch gekennzeichnet, dass** er die in SEQ ID NO: 2 oder in SEQ ID NO: 3 angegebene Sequenz umfasst.

13. Verwendung eines Primers nach Anspruch 12 oder eines Primerpaares nach den Ansprüchen 10 oder 11 zum spezifischen Nachweis von *M. tuberculosis.*

14. Hybridisierungssonde, **dadurch gekennzeichnet, dass** sie zum spezifischen Nachweis durch spezifische Hybridisierung des für *M. tuberculosis* spezifischen Polymorphismus, der an Position -215 in 5' zu 3' Leserichtung strangaufwärts des Translationsstartcodons GTG des narGRJI-Nitratreduktase-Operons lokalisiert ist, geeignet ist.

15. Hybridisierungssonden-Paar, **dadurch gekennzeichnet, dass** es zum spezifischen Nachweis durch spezifische Hybridisierung des für *M. tuberculosis* spezifischen Polymorphismus, der an Position -215 in 5' zu 3' Leserichtung strangaufwärts des Translationsstartcodons GTG des narGHJI-Nitratreduktase-Operons lokalisiert ist, geeignet ist.

16. Hybridisierungssonden-Paar nach Anspruch 15, **dadurch gekennzeichnet, dass** die Sonden die in Sequenz ID NO: 4 und SEQ ID NO: 5 angegebenen Sequenzen oder die komplementären Sequenzen davon umfassen.

17. Hybridisierungssonden-Paar nach Anspruch 15, **dadurch gekennzeichnet, dass** die Sonden die in Sequenz ID NO: 4 und SEQ ID NO: 6 angegebenen Sequenzen oder die komplementären Sequenzen davon umfassen.

18. Hybridisierungssonde, **dadurch gekennzeichnet, dass** sie die in SEQ ID NO: 4, SEQ ID NO: 5 oder SEQ ID NO: 6 angegebene Sequenz oder die komplementäre Sequenz davon umfasst.

19. Verwendung einer Hybridisierungssonde nach Anspruch 18 oder eines Hybridisierungssondenpaares nach den Ansprüchen 15 bis 17 zum spezifischen Nachweis von *M. tuberculosis.*

20. Verfahren nach Anspruch 1 umfassend die Schritte
a) Extraktion von mikrobieller DNA aus klinischem Material,
b) Amplifikation des DNA-Abschnitts aus der extrahierten DNA,
c) Detektion der spezifischen Hybridisierung des amplifizierten DNA-Abschnitts mit mindestens einer Hybridisierungssonde, welche die Nucleotidsequenz, ausgewählt aus der Gruppe bestehend aus SEQ ID NO: 5, der komplementären Sequenz zu SEQ ID NO: 5, SEQ ID NO: 6 und der komplementären Sequenz zu SEQ ID NO: 6, umfasst,
wobei der spezifische Nachweis von *M*. *tuberculosis* gegenüber M*. bovis, M. bovis* BCG, *M. africanum* und M. *microti* mittels Schmelzkurvenanalyse erfolgt.

21. Verfahren nach Anspruch 20, wobei in Schritt b) die Amplifikation mittels eines Primerpaares umfassend die Nucleotidsequenzen SEQ ID NO: 2 / SEQ ID NO: 3 erfolgt.

22. Verfahren nach einem der Ansprüche 20 oder 21, wobei in Schritt c) die spezifische Hybridisierung mit mindestens einem Paar markierter Hybridisierungssonden umfassend die Nucleotidsequenzen SEQ ID NO: 4 / SEQ ID NO: 5 oder das Paar der komplementären Sequenzen davon und/ oder die Nucleotidsequenzen SEQ ID NO: 4 / SEQ ID NO: 6 oder das Paar der komplementären Sequenzen davon erfolgt.

23. Verfahren nach einem der Ansprüche 20 bis 22, wobei die Vervielfachung der DNA-Fragmente mittels Polymerase-Kettenreaktion (PCR) bevorzugt als Echtzeit-PCR durchgeführt wird.

24. Verfahren nach einem der Ansprüche 20 bis 23, wobei die Schritte spezifischer Hybridisierung während oder nach der Amplifikation der DNA-Abschnitte erfolgen.

25. Verfahren nach einem der Ansprüche 20 bis 24, wobei die spezifische Hybridisierung und deren Detektion in der Echtzeit-PCR erfolgt.

26. Verfahren nach einem der Ansprüche 20 bis 25, wobei die Detektion der spezifischen Hybridisierung mittels Fluoreszenznachweis durchgeführt wird und wobei die markierten Hybridisierungssonden-Paare als Fluoreszenz-Resonanz-Energie-Transfer (FRET)-Paar ausgebildet sind.

27. Verfahren nach einem der Ansprüche 20 bis 26, wobei das klinische Material ausgewählt ist aus der Gruppe klinischer Proben bestehend aus Sputum, Bronchiallavage, Magensaft, Urin, Stuhl, Liquor, Knochenmark, Blut und Biopsien.

28. Oligonucleotid nach Anspruch 14, welches mit einer *M*. tuberculosis-spezifischen Promotorregion des narGHJI-Nitratreduktase-Operons spezifisch hybridisiert, umfassend die Nucleotidsequenz SEQ ID NO: 5, SEQ ID NO: 6 oder die jeweils komplementäre Sequenz davon.

29. Kit zum spezifischen Nachweis von *M. tuberculosis* in klinischem Material, umfassend
a) mindestens ein Primerpaar umfassend die Nucleotidsequenzen SEQ ID NO: 2 / SEQ ID NO: 3, und
b) mindestens ein Hybridisierungssonden-Paar umfassend die Nucleotidsequenzen SEQ ID NO: 4 / SEQ ID NO: 5 oder das Paar der komplementären Sequenzen davon und/oder mindestens ein Hybridisierungssonden-Paar umfassend die Nucleotidsequenzen SEQ ID NO: 4 / SEQ ID NO: 6 oder das Paar der komplementären Sequenzen davon.

30. Verwendung mindestens eines *M*. *tuberculosis*-spezifischen DNA-Polymorphismus in der Promotorregion des narGHJI-Nitratreduktase-Operons von Mykobakterien, enthaltend die Nucleinsäuresequenz dargestellt in SEQ ID NO: 6 oder die komplementäre Sequenz davon, zum spezifischen Nachweis einer Infektion mit *M. tuberculosis.*

## Claims

1. Method for the specific detection of *Mycobacterium tuberculosis (M. tuberculosis)* in clinical material, comprising steps wherein
microbial DNA is extracted from the clinical material,
at least one DNA fragment of the nitrate reductase operon of mycobacteria, that is the of the *narGHJI* operon encoding nitrate reductase, is amplified from the extracted DNA, wherein the amplified DNA fragment or one of the amplified DNA fragments comprises a target region comprising at least one DNA polymorphism specific for *M. tuberculosis* which is located in position -215 in the 5' to 3' direction of reading upstream of the translation start codon GTG of the *narGHJI* operon,
wherein the specific hybridization of the at least one amplified DNA fragment with at least one hybridization probe is detected.

2. Method according to claim 1 wherein the nucleic acid amplification is carried out by PCR, NASBA, SDA or LCR.

3. Method according to claim 2, wherein the PCR is a real time PCR.

4. Method according to claims 1 to 3, wherein the detection of the polymorphism specific for *M. tuberculosis* is carried out by the specific hybridization of one or several probes.

5. Method according to claims 1 to 4, wherein the pair of primers comprises at least one of the sequences having SEQ ID NO: 2 and SEQ ID NO:3.

6. Method according to claims 1 to 4, wherein the amplified DNA segment has a length of 155 nucleotides and the pair of primers comprises the sequences having SEQ ID NO: 2 and SEQ ID NO:3.

7. Method according to claims 1 to 6 wherein the detection of the polymorphism specific for *M. tuberculosis* takes place by means of pairs of labelled hybridization probes, one probe being labelled at its 3' end, and the other probe being labelled at its 5' end, and the probes binding specifically to the amplificate in such a way that a fluorescence resonance energy transfer (FRET) is possible.

8. Method according to claim 7, wherein the probe pair comprises the sequences having SEQ ID NO: 4 and 5 or the complementary sequences thereof and/or the sequences with SEQ ID NO: 4 and 6 or the complementary sequences thereof.

9. Method according to claims 1 to 8, wherein the clinical material is selected from the group of clinical samples consisting of sputum, bronchial lavage, gastric juice, urine, faeces, liquor, bone marrow, blood, biopsies and culture isolates.

10. Primer pair suitable for the amplification of a DNA segment comprising a segment from the area of the *narGHJI* nitrate reductase operon, wherein the DNA segment comprises position -215 in the 5' to 3' direction of reading upstream of the translation start codon GTG of the *narGHJI* nitrate reductase operon, wherein at least one primer comprises the sequence indicated in SEQ ID NO:2 or SEQ ID NO:3.

11. Primer pair according to claim 10, wherein the primers comprise the sequences indicated in SEQ ID NO: 2 and in SEQ ID NO: 3.

12. Primer comprising the sequence indicated in SEQ ID NO: 2 or in SEQ ID NO: 3.

13. Use of a primer according to claim 12 or a primer pair according to claims 10 or 11 for the specific detection of *M. tuberculosis.*

14. Hybridization probe suitable for the specific detection of the polymorphism specific for *M. tuberculosis* which is located in position -215 in the 5' to 3' direction of reading upstream of the translation start codon GTG of the *narGHJI* nitrate reductase operon, wherein the detection is by specific hybridization.

15. Pair of hybridization probes suitable for the specific detection of the polymorphism specific for *M. tuberculosis* which is located in position -215 in the 5' to 3' direction of reading upstream of the translation start codon GTG of the *narGHJI* nitrate reductase operon, wherein the detection is by specific hybridization.

16. Pair of hybridization probes according to claim 15, wherein the probes comprise the sequences indicated in SEQ ID NO: 4 and SEQ ID NO: 5 or the complementary sequences thereof.

17. Pair of hybridization probes according to claim 15, wherein the probes comprise the sequences indicated in SEQ ID NO: 4 and SEQ ID NO: 6 or the complementary sequences thereof.

18. Hybridization probe comprising the sequence indicated in SEQ ID NO: 4, SEQ ID NO: 5 or SEQ ID NO: 6 or the complementary sequence thereof.

19. Use of a hybridization probe according to claim 18 or a hybridization probe pair according to claims 15 to 17 for the specific detection of *M. tuberculosis.*

20. Method according to claim 1, comprising steps comprising
a) extraction of microbial DNA from clinical material,
b) amplification of at least one DNA fragment from the extracted DNA,
c) detection of the specific hybridization of the amplified DNA fragment with at least one hybridization probe which comprises the nucleotide sequence selected from the group consisting of SEQ ID NO: 5, the sequence complementary to SEQ ID NO: 5, SEQ ID NO: 6, and the sequence complementary to SEQ ID NO: 6,
wherein the specific detection of *M. tuberculosis* vis-à-vis *M. bovis*, *M. bovis* BCG, *M. africanum* and *M. microti* is carried out by melting curve analysis.

21. Method according to claim 20, wherein the amplification taking place in step b) is carried out by means of a primer pair comprising the nucleotide sequences SEQ ID NO: 2 / SEQ ID NO: 3.

22. Method according to one of claims 20 or 21, wherein the specific hybridization taking place in step c) is carried out with at least one pair of labelled hybridization probes comprising the nucleotide sequences SEQ ID NO: 4 / SEQ ID NO: 5 or the pair of complementary sequences thereof and/or the nucleotide sequences SEQ ID NO: 4 / SEQ ID NO: 6 or the pair of complementary sequences thereof.

23. Method according to one of claims 20 to 22, wherein the multiplication of the DNA fragments by polymerase chain reaction (PCR) is preferably carried out by real time PCR.

24. Method according to one of claims 20 to 23, wherein the steps of specific hybridization take place during or after the amplification of the DNA fragments.

25. Method according to one of claims 20 to 24, wherein the specific hybridization and its detection is carried out in real time PCR.

26. Method according to one of claims 20 to 25, wherein the detection of the specific hybridization is carried out by fluorescence detection, and wherein the labelled hybridization probe pairs are generated as fluorescence resonance energy transfer (FRET) pair.

27. Method according to one of claims 20 to 26, wherein the clinical material is selected from the group of clinical samples consisting of sputum, bronchial lavage, gastric juice, urine, faeces, liquor, bone marrow, blood and biopsies.

28. Oligonucleotide which specifically hybridizes with a *M. tuberculosis*-specific promoter region of the *narGHJI* nitrate reductase operon, comprising the nucleotide sequence SEQ ID NO: 5, SEQ ID NO: 6, or the respective complementary sequence thereof.

29. Kit for the specific detection of *Mycobacterium tuberculosis* comprising
a) at least one primer pair comprising the nucleotide sequences SEQ ID NO: 2 / SEQ ID NO: 3 and
b) at least one hybridization probe pair comprising the nucleotide sequences SEQ ID NO: 4 / SEQ ID NO: 5 or the pair of complementary sequences thereof and/or at least one hybridization probe pair comprising the nucleotide sequences SEQ ID NO: 4 / SEQ ID NO: 6 or the pair of complementary sequences thereof.

30. Use of at least one *M. tuberculosis*-specific DNA polymorphism in the promoter region of the *narGHJI* nitrate reductase operon of mycobacteria, containing the nucleic acid sequence represented in SEQ ID NO: 6 or the complementary sequence thereof, for the specific detection of an infection with *M. tuberculosis.*

## Revendications

1. Procédé de détection spécifique de *Mycobacterium tuberculosis (M. tuberculosis)* dans un matériau clinique, dans lequel
on extrait de l'ADN microbien du matériau clinique,
à partir de cet ADN extrait, on amplifie au moins un fragment d'ADN de l'opéron nitrate réductase des mycobactéries, c'est-à-dire l'opéron narGHJI qui code la nitrate réductase, étant entendu que le fragment d'ADN amplifié ou l'un des fragments d'ADN amplifiés contient une région cible qui comprend au moins un polymorphisme d'ADN spécifique de *M. tuberculosis,* qui est localisé en position -215, en amont du codon de début de traduction GTG de l'opéron narGHJI dans le sens de lecture 5' → 3',
et l'on détecte l'hybridation spécifique dudit fragment d'ADN amplifié au nombre d'au moins un avec au moins une sonde d'hybridation.

2. Procédé conforme à la revendication 1, **caractérisé en ce que** l'amplification de l'acide nucléique est réalisée par la technique PCR, NASBA, SDA ou LCR.

3. Procédé conforme à la revendication 2, **caractérisé en ce que** la technique PCR est une PCR en temps réel.

4. Procédé conforme à l'une des revendications 1 à 3, **caractérisé en ce que** la détection du polymorphisme spécifique de *M. tuberculosis* est réalisée par hybridation spécifique d'une ou de plusieurs sonde(s).

5. Procédé conforme à l'une des revendications 1 à 4, **caractérisé en ce que** la paire d'amorces comprend au moins l'une des séquences données en tant que Séquence N° 2 et Séquence N° 3.

6. Procédé conforme à l'une des revendications 1 à 4, **caractérisé en ce que** le segment d'ADN amplifié est long de 155 nucléotides et **en ce que** la paire d'amorces comprend les séquences données en tant que Séquence N° 2 et Séquence N° 3.

7. Procédé conforme à l'une des revendications 1 à 6, **caractérisé en ce que** la détection du polymorphisme spécifique de *M. tuberculosis* est réalisée à l'aide de paires de sondes d'hybridation marquées dont une sonde est marquée à son extrémité 3' et l'autre sonde est marquée à son extrémité 5', et les sondes se lient de manière spécifique au produit d'amplification de telle sorte qu'un FRET (transfert d'énergie de fluorescence par résonance) est rendu possible.

8. Procédé conforme à la revendication 7, **caractérisé en ce que** la paire de sondes comprend les séquences données en tant que Séquence N° 4 et Séquence N° 5, ou les séquences complémentaires de celles-ci, et/ou les séquences données en tant que Séquence N° 4 et Séquence N° 6, ou les séquences complémentaires de celles-ci.

9. Procédé conforme à l'une des revendications 1 à 8, **caractérisé en ce que** le matériau clinique est choisi dans l'ensemble d'échantillons cliniques constitué par un crachat, un liquide de lavage des bronches, du suc gastrique, de l'urine, des selles, du liquide cérébro-spinal, de la moelle osseuse, du sang, une biopsie ou un isolat de culture.

10. Paire d'amorces, appropriée pour l'amplification d'un segment d'ADN qui comprend un segment issu du domaine de l'opéron nitrate réductase narGHJI, lequel segment d'ADN comprend la position -215, en amont du codon de début de traduction GTG de l'opéron narGHJI dans le sens de lecture 5' → 3', **caractérisée en ce qu'**au moins l'une de ces amorces comprend la séquence indiquée en tant que Séquence N° 2 ou Séquence N° 3.

11. Paire d'amorces conforme à la revendication 10, **caractérisée en ce que** les amorces comprennent les séquences indiquées en tant que Séquence N° 2 et Séquence N° 3.

12. Amorce, **caractérisée en ce qu'**elle comprend la séquence indiquée en tant que Séquence N° 2 ou Séquence N° 3.

13. Utilisation d'une amorce conforme à la revendication 12 ou d'une paire d'amorces conforme à la revendication 10 ou 11 en vue de détecter spécifiquement *M. tuberculosis.*

14. Sonde d'hybridation, **caractérisée en ce qu'**elle est appropriée pour la détection spécifique, par hybridation spécifique, du polymorphisme spécifique de *M. tuberculosis* localisé en position -215, en amont du codon de début de traduction GTG de l'opéron nitrate réductase narGHJI dans le sens de lecture 5' → 3'.

15. Paire de sondes d'hybridation, **caractérisée en ce qu'**elle est appropriée pour la détection spécifique, par hybridation spécifique, du polymorphisme spécifique de *M. tuberculosis* localisé en position -215, en amont du codon de début de traduction GTG de l'opéron nitrate réductase narGHJI dans le sens de lecture 5' → 3'.

16. Paire de sondes d'hybridation, conforme à la revendication 15, **caractérisée en ce que** les sondes comprennent les séquences indiquées en tant que Séquence N° 4 et Séquence N° 5, ou les séquences complémentaires de celles-ci.

17. Paire de sondes d'hybridation, conforme à la revendication 15, **caractérisée en ce que** les sondes comprennent les séquences indiquées en tant que Séquence N° 4 et Séquence N° 6, ou les séquences complémentaires de celles-ci.

18. Sonde d'hybridation, **caractérisée en ce qu'**elle comprend la séquence indiquée en tant que Séquence N° 4, Séquence N° 5 ou Séquence N° 6, ou une séquence complémentaire de l'une de celles-ci.

19. Utilisation d'une sonde d'hybridation conforme à la revendication 18, ou d'une paire de sondes d'hybridation conforme à l'une des revendications 15 à 17, en vue de détecter spécifiquement *M*. *tuberculosis.*

20. Procédé conforme à la revendication 1, lequel procédé comporte les étapes suivantes :
a) extraction d'ADN microbien du matériau clinique ;
b) amplification du segment d'ADN issu de l'ADN extrait ;
c) détection de l'hybridation spécifique du segment d'ADN amplifié avec au moins une sonde d'hybridation qui comprend une séquence de nucléotides choisie dans l'ensemble constitué par la Séquence N° 5, la séquence complémentaire de la Séquence N° 5, la Séquence N° 6, et la séquence complémentaire de la Séquence N° 6 ;
et dans lequel la détection spécifique de *M. tuberculosis* par rapport à *M. bovis, M. bovis* BCG, *M. africanum* et *M. microti* est effectuée par analyse de la courbe de fusion.

21. Procédé conforme à la revendication 20, dans lequel, dans l'étape (b), on effectue l'amplification au moyen d'une paire d'amorces comprenant les séquences de nucléotides données en tant que Séquence N° 2 / Séquence N° 3.

22. Procédé conforme à la revendication 20 ou 21, dans lequel, dans l'étape (c), on réalise l'hybridation spécifique avec au moins une paire de sondes d'hybridation marquées comprenant les séquences de nucléotides données en tant que Séquence N° 4 / Séquence N° 5 ou la paire de séquences complémentaires de celles-ci, et/ou les séquences de nucléotides données en tant que Séquence N° 4 / Séquence N° 6 ou la paire de séquences complémentaires de celles-ci.

23. Procédé conforme à l'une des revendications 20 à 22, dans lequel la multiplication des fragments d'ADN par réaction en chaîne de polymérase (PCR) est réalisée de préférence en PCR en temps réel.

24. Procédé conforme à l'une des revendications 20 à 23, dans lequel les étapes d'hybridation spécifique ont lieu pendant ou après l'amplification des segments d'ADN.

25. Procédé conforme à l'une des revendications 20 à 24, dans lequel l'hybridation spécifique et sa détection ont lieu par PCR en temps réel.

26. Procédé conforme à l'une des revendications 20 à 25, dans lequel la détection de l'hybridation spécifique est effectuée par détection de fluorescence et les paires de sondes d'hybridation marquées sont construites en tant que paires de sondes FRET (transfert d'énergie de fluorescence par résonance).

27. Procédé conforme à l'une des revendications 20 à 26, pour lequel le matériau clinique est choisi dans l'ensemble d'échantillons cliniques constitué par un crachat, un liquide de lavage des bronches, du suc gastrique, de l'urine, des selles, du liquide cérébro-spinal, de la moelle osseuse, du sang et une biopsie.

28. Oligonucléotide conforme à la revendication 14, qui s'hybride spécifiquement avec une région promoteur de l'opéron nitrate réductase narGHJI, spécifique de *M. tuberculosis,* comprenant la séquence de nucléotides donnée en tant que Séquence N° 5 ou Séquence N° 6, ou la séquence complémentaire de l'une de celles-ci.

29. Trousse conçue pour la détection spécifique de *M. tuberculosis* dans un matériau clinique, laquelle trousse comprend :
a) au moins une paire d'amorces comprenant les séquences de nucléotides données en tant que Séquence N° 2 / Séquence N° 3 ;
b) et au moins une paire de sondes d'hybridation comprenant les séquences de nucléotides données en tant que Séquence N° 4 / Séquence N° 5 ou la paire de séquences complémentaires de celles-ci, et/ou au moins une paire de sondes d'hybridation comprenant les séquences de nucléotides données en tant que Séquence N° 4 / Séquence N° 6 ou la paire de séquences complémentaires de celles-ci.

30. Utilisation d'au moins un polymorphisme d'ADN spécifique de *M. tuberculosis,* situé dans le région promoteur de l'opéron nitrate réductase narGHJI des mycobactéries, contenant la séquence d'acide nucléique présentée en tant que Séquence N° 6 ou la séquence complémentaire de celle-ci, en vue de la détection spécifique d'une infection par *M. tuberculosis.*


### SEQUENZPROTOKOLL

<110> Artus - Gesellschaft fuer molekularbiologische Diagnostik und Entwicklung mbH
<120> Verfahren und Kit zum spezifischen Nachweis von M. tuberculosis
<130> P 65555
<160> 6
<170> PatentIn version 3.1
<210> 1
   <211> 3002
   <212> DNA
   <213> Mycobacterium tuberculosis
<400> 1
   ctcgggtgtc aagttgacgc cggcgattac cgctgtctac ctcgtcggcg ttcggcggtt      60
   gcatgcggcc gcattttcgg tggtcgtgtt ccttgccacc gtcggcgtgt cgctactggt     120
   cgtcggcgat gaagcccgct actacttcac cgacctgttg ggcgacgcag gccgggttgg     180
   gcccatcgcc acctccttca atcaatcctg gcgcggcgcg atttcccgga ttctcggtca     240
   cgacgccggt tttggtccgc tggttctggc tgcgatcgcc agtacggcgg tattggccat     300
   cctggcctgg cgtgcgctcg acaggtccga tcggctgggc aaactattgg tggtcgagtt     360
   gttcggcctg ctgctctcgc cgatctcctg gactcaccac tgggtgtggc tagtgccgct     420
   gatgatctgg ctgattgacg ggccagcgcg tgagcgcccg ggcgcccgga ttttgggctg     480
   gggctggttg gtgttgacca tcgtcggcgt gccgtggttg ctgagctttg ctcaaccgag     540
   catctggcaa atcggccggc cgtggtattt ggcctgggcc ggtctggtct acgtggtggc     600
   gacgctggcg accttgggct ggatcgccgc ctccgagcgt tacgtgcgca ttcggccgcg     660
   gcgcatggcc aattaggccc caaacattgc gtcgatatcg tgcgccatcg caatgtcgtt     720
   ttccgtgata ccacctaccg catgcgtaac cagcgcgaaa gttactgttc gccaacggat     780
   atcgatgtcc ggatgatgat ttacctcctc ggctcgctcg gccacccggc gtacggcgtc     840
   gataccggcc ataaacgtcg gaaacttgat tgacctacgc aggacaccac cggcgcgctg     900
   ccagccgttg aggtcgtgca gtgcggcgtc gacctgctca tccgttaaca cagccatacc     960
   tcgacggtat accgtcacag gtcatgctga atcagatcgt ggttgccgga gccatcgtcc    1020
   gcggttgcac ggtcttggtg gcgcaacgcg ttcggccacc ggagttggcg ggtcgttggg    1080
   aacttcccgg cggtaaggtc gccgccggcg aaaccgagcg cgccgcgctg gcccgagagc    1140
   tcgccgaaga actgggactc gaggtcgccg acctcgcggt gggcgaccgt gtgggcgacg    1200
   atattgcgtt gaacggcacg acgacgctgc gggcctatcg cgtgcatctg cttggcggcg    1260
   aaccgcgtgc gcgtgaccac cgggcgctgt gctgggtgac ggcggccgaa ctgcacgatg    1320
   tcgactgggt accagccgac cgcggctgga ttgcggacct ggcgcgaacc ctcaacgggt    1380
   ccgccgcaga tgtccaccgt cgctgttagg aaaccgacgg tgtggttgac ggtggccgcc    1440
   gtcaacttgg ttagaacaac gtgacaaaac gttaacttgg gtttgcatgc ccgtagcgat    1500
   tacgatggtt ttctggacgc gtggcgacaa cttccgggca ggacgctgac gcccatccat    1560
   cgagataccc gatgttgacg agaggggtcc ccgacccggc ggaccggggc ttgacgggcg    1620
   caatgcggcg cggccggcca gcccgtaacg tccagcgagt gcggtcgcgc gccgacggcc    1680
   cggccccaca ccgctcatga cgaggagggt catcccgtga ccgttacacc tcacgtcggt    1740
   ggaccgctcg aagagctgct ggagcgcagc gggcgcttct tcaccccagg tgagttctcg    1800
   gccgacctgc gcaccgtaac ccggcgcggc ggccgcgaag gtgacgtgtt ctaccgcgat    1860
   cggtggagtc acgacaaagt ggtccgatcc acgcacggag tcaactgcac cggatcctgc    1920
   tcatggaaga tctacgtcaa agacgggatc atcacctggg aaacccagca gaccgactac    1980
   ccgtcggtgg gcccggaccg gcccgaatac gagccacgag gttgtccccg tggcgcgtcg    2040
   ttctcctggt acagctattc gccgacgcgg gtgcgctatc cgtatgcccg gggcgtgctg    2100
   gttgagatgt accgggaagc caagacccgc ctgggcgacc cggtgctggc gtgggccgac    2160
   attcaggcgg atcccgagcg cagacgccgc tatcaacagg cccgcggcaa gggtgggctg    2220
   gtccgggtga gctgggccga ggccagcgag atggtggccg ccgcccacgt gcacaccatc    2280
   aagacatacg gcccggaccg ggtcgccggc ttctcgccga ttccggcgat gtcaatggtc    2340
   agccatgccg cggggtcccg gttcgtggag ctgatcggcg gcgtgatgac gtcgttctac    2400
   gactggtacg ccgacttgcc ggtggcctcg ccgcaggtgt tcggcgacca gaccgacgtg    2460
   cccgaatccg gcgactggtg ggatgcgtcg tatttggtca tgtggggctc caacgtcccg    2520
   atcacccgga cgcccgacgc acattggatg gcggaggccc gttaccgcgg cgctaaagtc    2580
   gttgtcgtca gcccggacta cgccgacaac accaagttcg ccgacgagtg ggtgcggtgc    2640
   gccgccggta ccgataccgc gctggcgatg gcgatgggcc acgtgatcct gtcggaatgt    2700
   tacgtccgta accaggttcc gttctttgtc gactatgtgc gccgctacac cgacctgccg    2760
   tttttgatca agttggaaaa gcggggcgac ctgctggttc ccggaaagtt cttgaccgcg    2820
   gccgacattg gtgaagaaag tgagaacgcg gcgttcaaac ccgccctgct ggatgagctt    2880
   acgaataccg ttgtcgtgcc gcagggctca ctgggattcc gtttcggtga ggacggtgtt    2940
   gggaagtgga acctggacct gggttcggtg gtgccggcgc taagtgtgga gatggacaag    3000
   gc                                                                  3002
<210> 2
   <211> 19
   <212> DNA
   <213> Mycobacterium
<400> 2
   aaccgacggt gtggttgac 19
<210> 3
   <211> 19
   <212> DNA
   <213> Mycobacterium
<400> 3
   atctcgatgg atgggcgtc 19
<210> 4
   <211> 22
   <212> DNA
   <213> Mycobacterium
<400> 4
   gtcgccacgc gtccagaaaa cc 22
<210> 5
   <211> 18
   <212> DNA
   <213> Mycobacterium
<400> 5
   cgtgatcgct acgggcat 18
<210> 6
   <211> 19
   <212> DNA
   <213> Mycobacterium
<400> 6
   cgtaatcgct acgggcatg 19